# EUROPEAN PATENT SPECIFICATION

(11) **EP 4 363 861 B1**
(45) Date of publication and mention of the grant of the patent: **03.12.2025**
(21) Application number: 22761589.5
(22) Date of filing: 29.06.2022
(51) Int. Cl.: G01N 33/68

(54) **DIAGNOSTIC AND TREATMENT MONITORING BASED ON BLOOD-BRAIN BARRIER DISRUPTION**
DIAGNOSTISCHE UND BEHANDLUNGSÜBERWACHUNG AUF BASIS VON STÖRUNGEN DER BLUT-HIRN-SCHRANKE
SURVEILLANCE DE DIAGNOSTIC ET DE TRAITEMENT BASÉE SUR UNE RUPTURE DE LA BARRIÈRE HÉMATO-ENCÉPHALIQUE

(30) Priority: 01.07.2021 US 202163217543 P
(43) Date of publication of application: 08.05.2024
(73) Proprietor: Insightec Ltd., 39120 Tirat-Carmel (IL)
(72) Inventor: ACHROL, Achal, Singh, Miami, FL 33131 (US); LEVY, Yoav, Hinanit, Ot (IL)
(74) Representative: Dennemeyer & Associates S.A.
(86) International application number: PCT/IB2022/000376
(87) International publication number: WO 2023/275617

(56) References cited:
- US-A1- 2019 323 086
- MENG YING ET AL: "MR-guided focused ultrasound liquid biopsy enriches circulating biomarkers in patients with brain tumors", NEURO-ONCOLOGY, vol. 23, no. 10, 10 March 2021 (2021-03-10), US, pages 1789 - 1797, XP055978651, ISSN: 1522-8517, Retrieved from the Internet <URL:https://www.ncbi.nlm.nih.gov/pmc/articles/PMC8485448/pdf/noab057.pdf> DOI: 10.1093/neuonc/noab057
- PACIA CHRISTOPHER PHAM ET AL: "Feasibility and safety of focused ultrasound-enabled liquid biopsy in the brain of a porcine model", SCIENTIFIC REPORTS, vol. 10, no. 1, 1 December 2020 (2020-12-01), pages 7449, XP055978649, Retrieved from the Internet <URL:https://www.nature.com/articles/s41598-020-64440-3.pdf> DOI: 10.1038/s41598-020-64440-3
- ZHU LIFEI ET AL: "Focused Ultrasound-enabled Brain Tumor Liquid Biopsy", SCIENTIFIC REPORTS, vol. 8, no. 1, 26 April 2018 (2018-04-26), XP055978643, Retrieved from the Internet <URL:http://www.nature.com/articles/s41598-018-24516-7> DOI: 10.1038/s41598-018-24516-7
- ZHU LIFEI ET AL: "Focused ultrasound for safe and effective release of brain tumor biomarkers into the peripheral circulation", PLOS ONE, vol. 15, no. 6, 3 June 2020 (2020-06-03), pages 1 - 15, XP055978648, Retrieved from the Internet <URL:https://journals.plos.org/plosone/article/file?id=10.1371/journal.pone.0234182&type=printable> DOI: 10.1371/journal.pone.0234182
- SABBAGH ARIA ET AL: "Opening of the Blood-Brain Barrier Using Low-Intensity Pulsed Ultrasound Enhances Responses to Immunotherapy in Preclinical Glioma Models", CLINICAL CANCER RESEARCH, vol. 27, no. 15, 24 May 2021 (2021-05-24), US, pages 4325 - 4337, XP055978657, ISSN: 1078-0432, Retrieved from the Internet <URL:https://watermark.silverchair.com/4325.pdf?token=AQECAHi208BE49Ooan9kkhW_Ercy7Dm3ZL_9Cf3qfKAc485ysgAAAtUwggLRBgkqhkiG9w0BBwagggLCMIICvgIBADCCArcGCSqGSIb3DQEHATAeBglghkgBZQMEAS4wEQQMflB-XsFmqjkEUA3-AgEQgIICiG3zm_88wEsYl151wfd9KLEG7PPbwdDZJk6g6OA5ne2RuoKNuZiAUuEyRKhAFKWCBZ-dEKDj_6uPBp84sJ74ypyfDcLZjmi> DOI: 10.1158/1078-0432.CCR-20-3760
- PRADA FRANCESCO ET AL: "Applications of Focused Ultrasound in Cerebrovascular Diseases and Brain Tumors", NEUROTHERAPEUTICS, SPRINGER INTERNATIONAL PUBLISHING, CHAM, vol. 16, no. 1, 7 November 2018 (2018-11-07), pages 67 - 87, XP036927102, ISSN: 1933-7213, [retrieved on 20181107], DOI: 10.1007/S13311-018-00683-3
- BING K F ET AL: "Blood-Brain Barrier (BBB) Disruption Using a Diagnostic Ultrasound Scanner and Definity(R) in Mice", ULTRASOUND IN MEDICINE AND BIOLOGY, NEW YORK, NY, US, vol. 35, no. 8, 1 August 2009 (2009-08-01), pages 1298 - 1308, XP026321831, ISSN: 0301-5629, [retrieved on 20090709], DOI: 10.1016/J.ULTRASMEDBIO.2009.03.012
- TAO SUN ET AL: "Acoustic cavitation-based monitoring of the reversibility and permeability of ultrasound-induced blood-brain barrier opening", PHYSICS IN MEDICINE AND BIOLOGY, INSTITUTE OF PHYSICS PUBLISHING, BRISTOL GB, vol. 60, no. 23, 12 November 2015 (2015-11-12), pages 9079 - 9094, XP020293692, ISSN: 0031-9155, [retrieved on 20151112], DOI: 10.1088/0031-9155/60/23/9079
- IDBAIH AHMED ET AL: "Safety and Feasibility of Repeated and Transient Blood-Brain Barrier Disruption by Pulsed Ultrasound in Patients with Recurrent Glioblastoma", CLINICAL CANCER RESEARCH, vol. 25, no. 13, 1 July 2019 (2019-07-01), US, pages 3793 - 3801, XP093025645, ISSN: 1078-0432, Retrieved from the Internet <URL:https://watermark.silverchair.com/3793.pdf?token=AQECAHi208BE49Ooan9kkhW_Ercy7Dm3ZL_9Cf3qfKAc485ysgAAAzgwggM0BgkqhkiG9w0BBwagggMlMIIDIQIBADCCAxoGCSqGSIb3DQEHATAeBglghkgBZQMEAS4wEQQMsRiwgnSPn24fACR5AgEQgIIC61hTC09iugnVQSIXmLT0WBE7mTpNLVtjsxb5VwgPuQcx456uADuvSaTd3I8gnXzkdPPolfHIbxfWs0FPNQlWj8XoisKxHWd> DOI: 10.1158/1078-0432.CCR-18-3643
- ZHANG DANIEL Y. ET AL: "Ultrasound-mediated Delivery of Paclitaxel for Glioma: A Comparative Study of Distribution, Toxicity, and Efficacy of Albumin-bound Versus Cremophor Formulations", CLINICAL CANCER RESEARCH, vol. 26, no. 2, 15 January 2020 (2020-01-15), US, pages 477 - 486, XP093025647, ISSN: 1078-0432, Retrieved from the Internet <URL:https://watermark.silverchair.com/477.pdf?token=AQECAHi208BE49Ooan9kkhW_Ercy7Dm3ZL_9Cf3qfKAc485ysgAAAzMwggMvBgkqhkiG9w0BBwagggMgMIIDHAIBADCCAxUGCSqGSIb3DQEHATAeBglghkgBZQMEAS4wEQQMqwA0wSK1zLO6U5QlAgEQgIIC5uPT-AFO4mD2GwFLvdDZCaY24UN7tBnDE7KxrPDtDJkPtN7sRkwoxDtdBZwj7M_2P7r5hTfDBMwwMVs2VTJw_OoqkAuOL_s-> DOI: 10.1158/1078-0432.CCR-19-2182
- SONABEND ADAM M ET AL: "Repeated opening of the blood-brain barrier with the skull-implantable SonoCloud-9 (SC9) device: Phase 1 trial of nab-paclitaxel and SC9 in recurrent glioblastoma", JOURNAL OF CLINICAL ONCOLOGY, vol. 40, no. 16 supplement, 1 June 2022 (2022-06-01), pages 2016 - 2016, XP093025648, Retrieved from the Internet <URL:https://d32wbias3z7pxg.cloudfront.net/meeting/288/abstract/pdf/288-14351-207623.pdf>

## Description

A computer readable format copy of the Sequence Listing (Filename: "INS-124PC_ST25.txt"; Date created: June 22, 2022; File size: 4,096 bytes).

### BACKGROUND

Liquid biopsy involves the detection and analysis of pathology-derived material in blood without the need for invasive interventions such as open surgery. In cancer, it has the potential to provide a unifying platform for diagnosis, informing treatment selection by detecting resistant or sensitive tumor variants, and disease response monitoring. This approach has made strides for systemic cancers, with the development of several clinically approved assays for circulating tumor DNA ("ctDNA") and circulating tumor cells, among others. Its use in, *e.g.,* central nervous system (CNS) tumors, however, is severely limited by the blood-brain barrier ("BBB"), which prevents biomarker transit. Even with the altered blood-tumor barrier, there is substantial heterogeneity in permeability that can prevent representation of the tumor in circulation.

Similarly, diseases of the brain or central nervous system, e.g. Alzheimer's disease and Parkinson's disease, suffer from detection and diagnostic limitation due to the prevent of biomarker transit across the BBB and into the bloodstream.

Thus, there remains a significant need for improved compositions and methods that enable the sensitive detection of brain-derived biomarkers reflecting the pathology for clinical treatment and decision-making.

Related methods are described in Meng et al., "MR-guided focused ultrasound liquid biopsy enriches circulating biomarkers in patients with brain tumors", Neuro Oncol. 23(10):1789-1797; US 2019/323086; Pacia et al., "Feasibility and safety of focused ultrasound-enabled liquid biopsy in the brain of a porcine model", Sci Rep. 10: 7449; Zhu et al., "Focused Ultrasound-enabled Brain Tumor Liquid Biopsy", Sci Rep 8: 6553; Zhu et al., "Focused ultrasound for safe and effective release of brain tumor biomarkers into the peripheral circulation", PLoS One 15(6):e0234182; and Sabbagh et al., "Opening of the Blood-Brain Barrier Using Low-Intensity Pulsed Ultrasound Enhances Responses to Immunotherapy in Preclinical Glioma Models", Clin Cancer Res 27(15):4325-4337.

### SUMMARY

The invention provides a method of evaluating a subtype or mutational basis of a disease or disorder of the brain in a human patient, comprising: (a) contacting a plasma sample obtained from the human patient, the human patient having been exposed to an ultrasound beam across the cranium to cause disruption of the blood-brain barrier (BBB) and to permit the transit of one or more brain-derived biomarkers into the blood, with a detection agent to detect the presence, absence or level of the one or more brain-derived biomarkers, optionally wherein the human patient has been suspected of having the disease or disorder of the brain or at risk for developing a disease or disorder of the brain; and (b) determining subtype or mutational basis of the disease or disorder based on step (a).

The details of one or more examples of the disclosure are set forth in the description below. Other features or advantages of the present disclosure will be apparent from the following drawings, detailed description of several examples, and also from the dependent claims. The details of the disclosure are set forth in the accompanying description below. Although methods and materials similar or equivalent to those described herein can be used in the practice or testing of the present disclosure, illustrative methods and materials are now described. Other features, objects, and advantages of the disclosure will be apparent from the description and from the claims. In the specification and the claims, the singular forms also include the plural unless the context clearly dictates otherwise. Unless defined otherwise, all technical and scientific terms used herein have the same meaning as commonly understood by one of ordinary skill in the art to which this disclosure belongs.

### BRIEF DESCRIPTION OF THE DRAWING

The patent or application file contains at least one drawing executed in color.

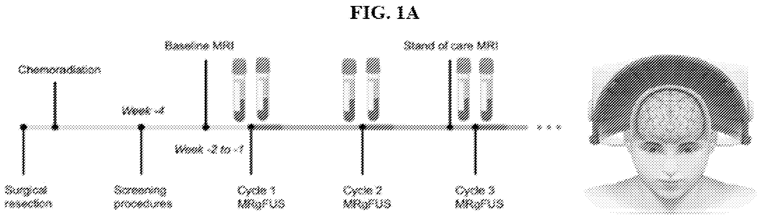

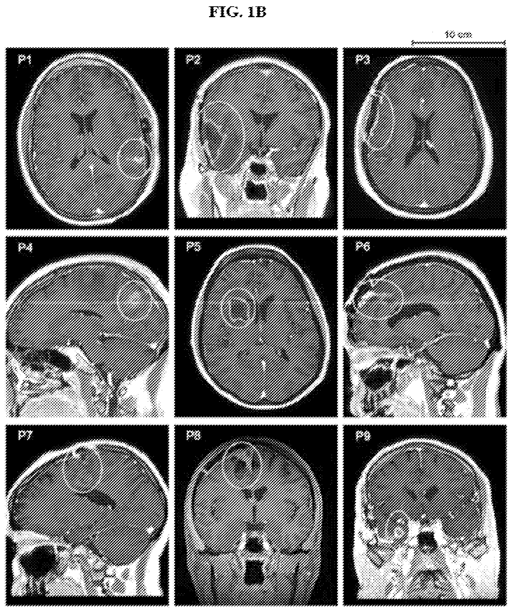

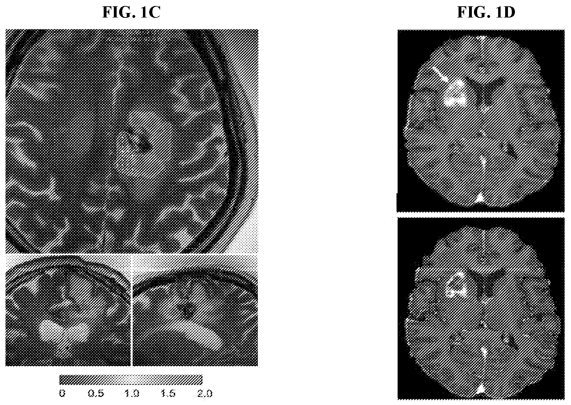

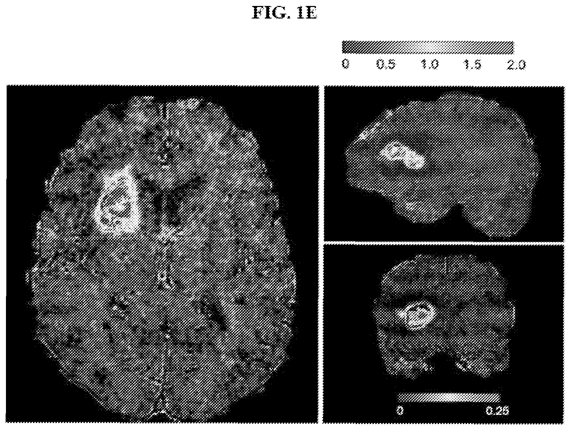

FIG. 1A, FIG. 1B, FIG. 1C, FIG. 1D, and FIG. 1E are images showing how opening of the blood-brain barrier in large brain regions is achieved with transcranial MR-guided focused ultrasound. FIG. 1A shows a diagram of a magnetic resonance guided focused ultrasound ("MRgFUS") device and study design with a blood-brain barrier opening ("BBBO") procedures overlapping adjuvant chemotherapy regimen in patients with glioblastoma after surgical resection and concurrent chemoradiation. Blood samples are collected immediately before and after sonication on the same day of the procedure. FIG. 1B shows images of the lesions (yellow circles) on baseline MRI for each patient. FIG. 1C shows images how ultrasound can be targeted specifically to an operator-defined contour. The green polygon represents one of many target contours (others not shown). The color map indicates the cavitation dose detected by device upon ultrasound delivery. In FIG. 1D, the top panel shows increased contrast extravasation in the areas of increased BBB permeability after MRgFUS for P5 (yellow arrow). The bottom panel of FIG. 1D shows partial restoration of the BBB integrity approximately 24 hours later, in comparison to the top panel and baseline image of the tumor in FIG. 1B. FIG. 1E shows images of intensity difference maps and further show increased BBB permeability in P5 within large regions spatially distributed in the tumor and tumor margins. Heterogeneous changes in contrast extravasation is partially due to the underlying tumor.

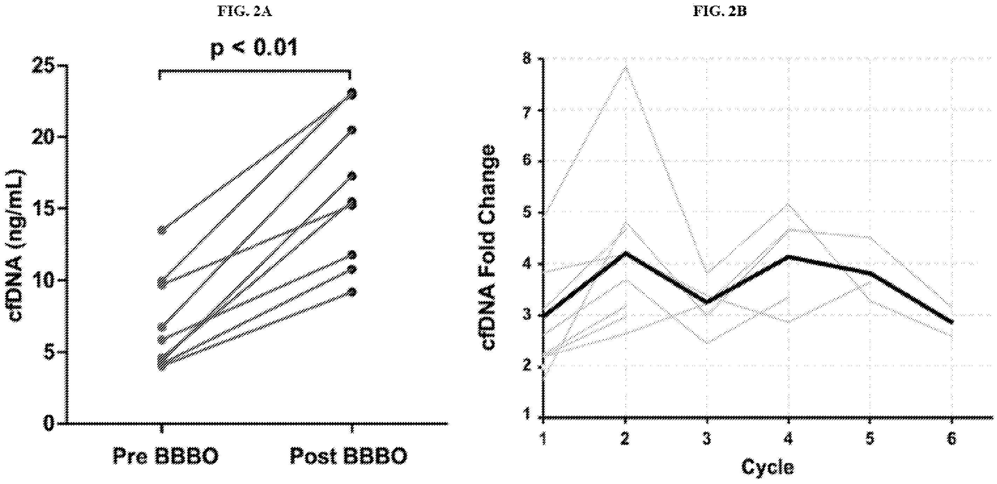

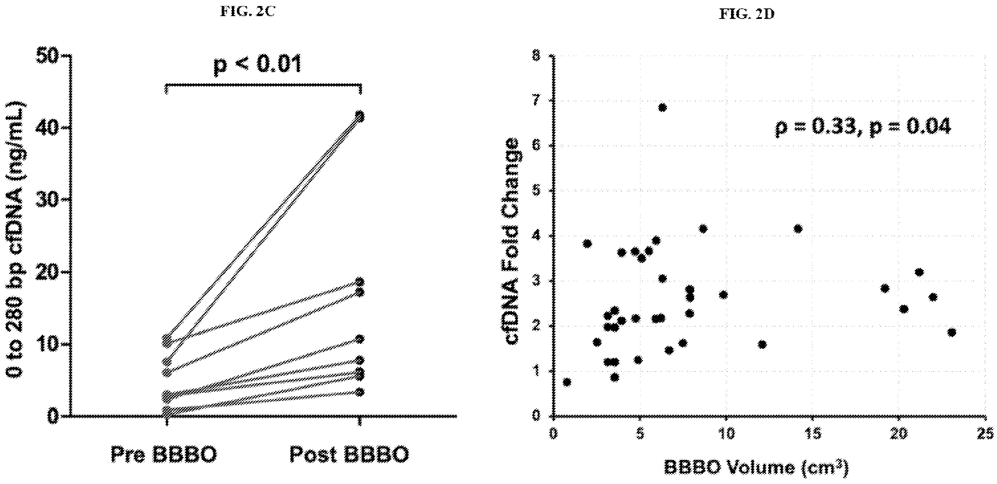

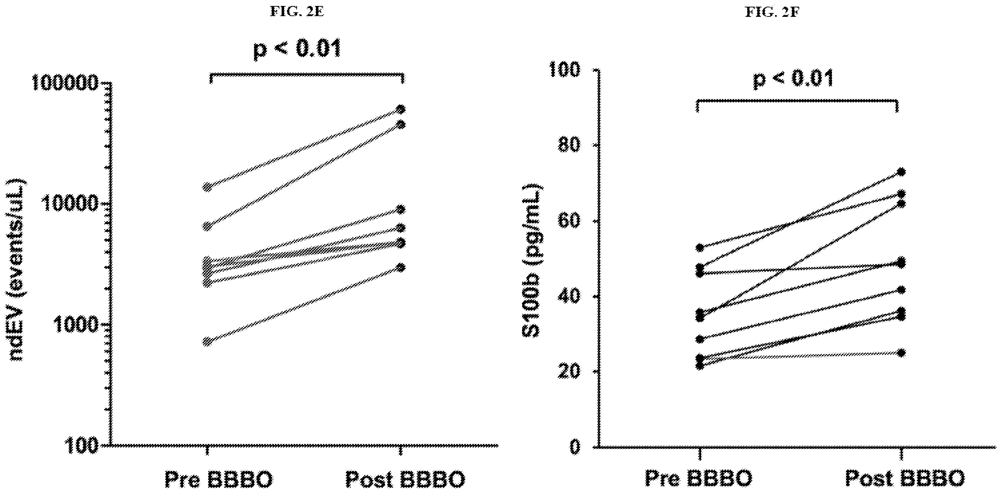

FIG. 2A, FIG. 2B, FIG. 2C, FIG. 2D, FIG. 2E, and FIG. 2F are graphs showing how transient blood-brain barrier opening enriches signal of circulating biomarkers. FIG. 2A is a graph showing how plasma cfDNA concentration was significantly enhanced after noninvasive MRgFUS BBBO. Each data point represents the mean cfDNA concentration for each patient. FIG. 2B is a graph showing the fold change in plasma cfDNA concentration, which is graphed over the course of adjuvant chemotherapy. Each gray line represents a single patient. The solid black line indicates the group mean at each cycle. FIG. 2C is a graph showing the concentration of single nucleosome length cfDNA and how it was also increased after BBBO. FIG. 2D is a graph showing the fold change in plasma cfDNA concentration at each cycle, which is plotted against the volume of BBBO, demonstrating a positive Spearman's correlation between the two variables. Each point represents the data at one cycle. FIG. 2E is graph showing L1CAM (also known as CD171), and NCAM double-positive extracellular vesicles are increased after BBBO. FIG. 2F is a graph showing how plasma S100b levels are significantly increased after BBBO. In FIG. 2C, FIG. 2E, and FIG. 2F, measurements were taken pre- and post-BBBO at only one cycle per patient, given availability of samples. In FIG. 2E, "ndEV" refers to "neuron-derived extracellular vesicle."

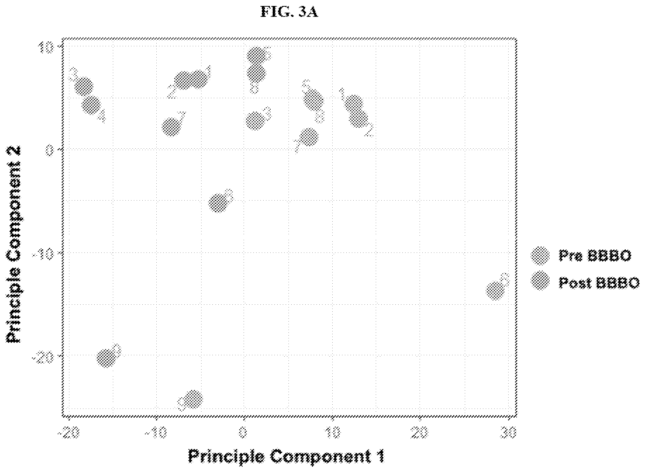

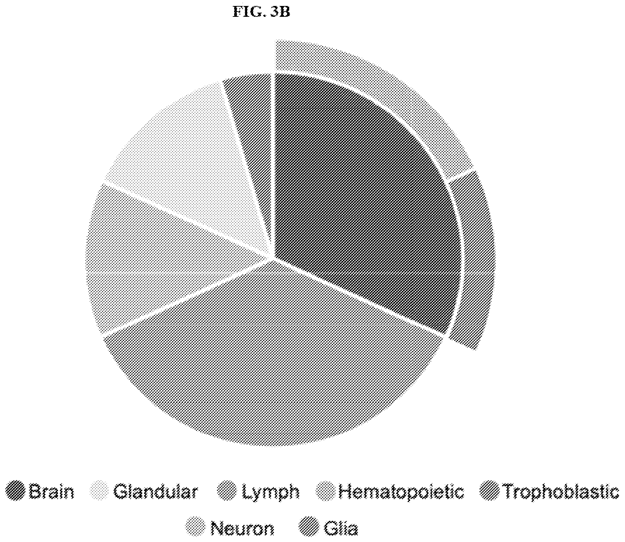

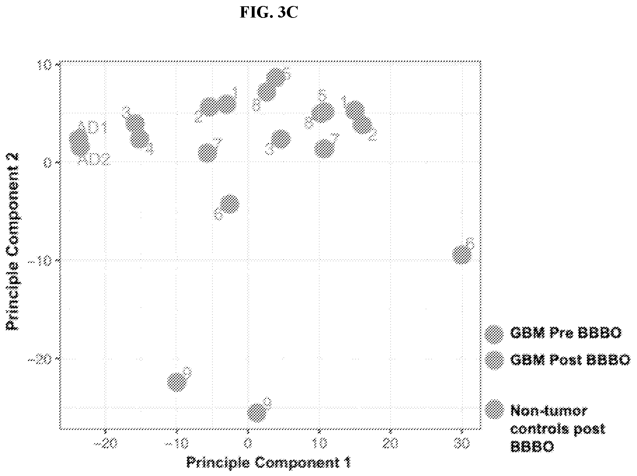

FIG. 3A, FIG. 3B, and FIG. 3C are graphs showing how post-BBBO cfDNA have distinctive disease-specific signature. FIG. 3A is a graph of principle component analysis showing a separation of the methylation signature of pre- and post-BBBO cfDNA. FIG. 3B is graph showing the result of a functional enrichment analysis of the differentially hypomethylated probe set based on the Human Protein Atlas. FIG. 3C is graph showing the results of a principle component analysis of methylation data with cfDNA from non-brain tumor patients post-BBBO as controls suggest disease-specific signatures. In FIG. 3C, "GBM" refers to glioblastoma.

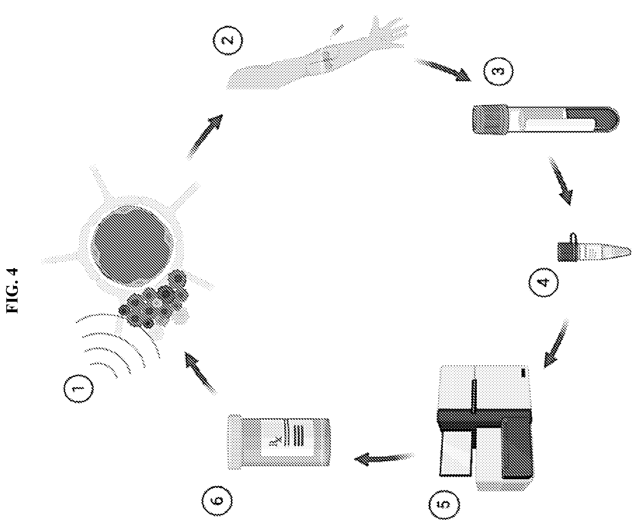

FIG. 4 is a schematic showing a MRgFUS-based platform for both enhanced drug delivery and liquid biopsy. FIG. 4 is a concept diagram of a MRgFUS-based platform for both enhancing drug delivery and liquid biopsy to deliver personalized medicine for patients with brain tumors. MRgFUS can be combined with repeated doses of anti-tumor therapy (1), each time providing an opportunity to collect blood samples containing analytes derived from the pathology in the sonicated areas (2). After pre-processing (3) and extraction for specific markers (4), commercially available techniques were used for processing (5, *e.g.,* droplet digital PCR). Biological changes in the pathology might inform a change in anti-tumor therapy (6).

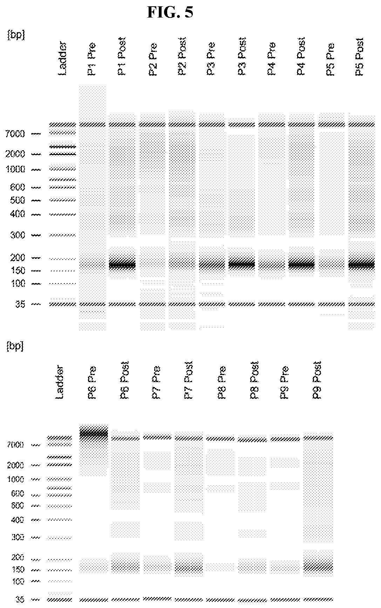

FIG. 5 shows two images of a DNA electrophoresis of nine paired pre-post BBBO cfDNA samples. As shown in FIG. 5, the plasma 0 to 280 bp fragment size cfDNA is increased in each patient after MRgFUS induced BBBO.

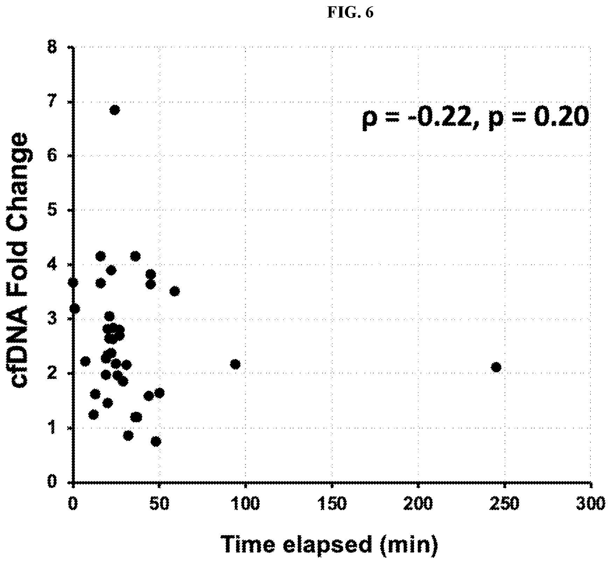

FIG. 6 is a graph showing the fold change in cfDNA compared to time elapsed from completion of sonication. In FIG. 6, "ρ" refers to Spearman's correlation.

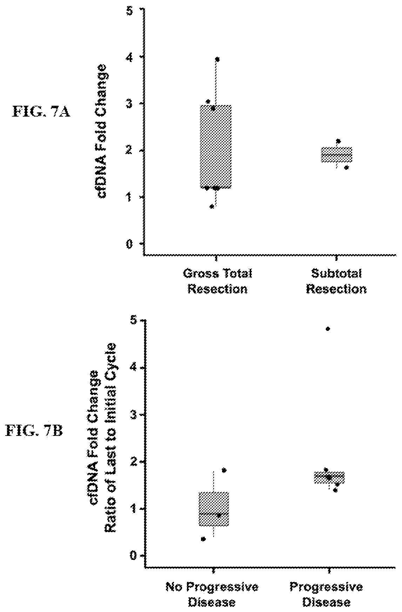

FIG. 7A and FIG. 7B are graphs showing how the MRgFUS induced enhancement in cfDNA concentration at the initial TMZ cycle is stratified by patients with or without residual tumor on MRI. FIG. 7A shows level of cfDNA enhancement in two patients with a subtotal resection.
FIG. 7B shows the fold change in plasma cfDNA at the last cycle for each patient normalized to their initial cycle, stratified by patients who did and did not have progressive disease by RANO Criteria.

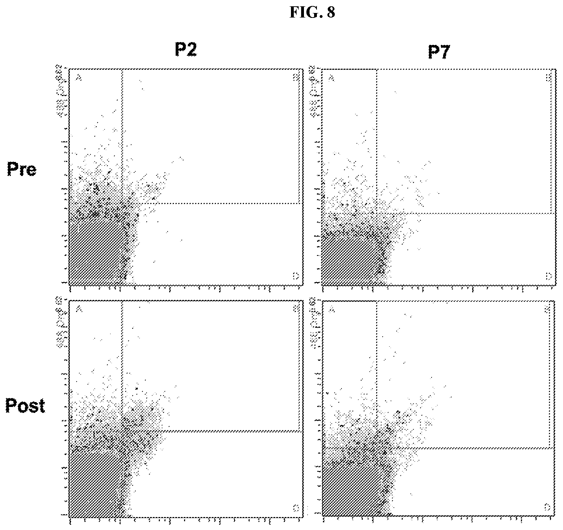

FIG. 8 is a graph showing nanoscale flow cytometry data. Nanoscale flow cytometry data of plasma samples from P2 and P7 in FIG. 8 shows the number of L1CAM (vertical axis) and NCAM (horizontal axis) double-positive particles (quadrant B) increases from pre- to post-BBBO.

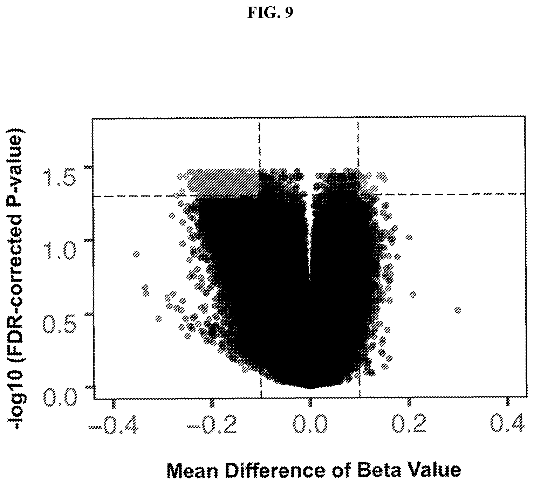

FIG. 9 is a graph showing a comparison of differentially methylated probes between post- vs pre-BBBO cfDNA. In FIG. 9, volcano plot shows differentially methylated probes between post-vs pre-BBBO are 95% more hypomethylated, consistent with a cancer signature. Dots to the left of 0.0 on the x axis of the graph indicate hypomethylated probes in post compared to pre-BBBO samples, and to the right of 0.0, hypermethylated probes. Red dots represent significantly differentially methylated probes that were identified by an absolute difference of mean beta value > 0.1 and FDR-corrected p < 0.05.

### DETAILED DESCRIPTION OF CERTAIN EMBODIMENTS

The present invention is based, in part, on the surprising discovery that ultrasound application to the brain enriches the signal of circulating brain-derived biomarkers, demonstrating the potential of the technology to support liquid biopsy for the brain. As disclosed herein, MRgFUS can non-invasively open the BBB, which normally limits therapeutic access to brain regions and also limits shedding of brain pathology-derived biomarkers into the bloodstream for liquid biopsy, and therefore focused ultrasound enhances circulating brain-derived biomarkers, supporting the feasibility of focused ultrasound to aid liquid biopsy.

Liquid biopsy is promising for detection, monitoring of response, and for detecting the recurrence of cancer. However, the blood-brain barrier ("BBB") limits the transit of biomarker, such as cell-free DNA (cfDNA), into the blood, and their detection by conventional assays. To address this hurdle, MRgFUS is an emerging technology that permits non-invasive access to brain pathology by inducing transient BBB opening (BBBO). MRI-guidance imparts spatially precise and flexible selection of sonicated brain regions.

Experiments and examples of the present disclosure, among other things, represent the first in-human demonstration that non-invasive, transient opening of the BBB using focused ultrasound technology can enrich circulating brain-derived biomarkers with potential applications in liquid biopsy. As disclosed herein, transcranial focused ultrasound can safely and open, e.g. transiently open, the BBB, providing an opportunity for less-invasive access to brain pathology. Furthermore, precise targeting achieved by present technologies ensures that materials (e.g., biomarkers) released into the blood via the BBB opening, e.g. transient BBB opening, are co-localized with, and thus likely to be associated with, brain pathology *(e.g.,* a tumor or other site of damage, *etc.).* Experiments and examples disclosed herein demonstrate, among other things, that focused ultrasound can enrich the signal of circulating brain-derived biomarkers to aid in liquid biopsy.

Provided is a method of evaluating a subtype or mutational basis of a disease or disorder of the brain in a human patient, comprising: (a) contacting a plasma sample obtained from the human patient, the human patient having been exposed to an ultrasound beam across the cranium to cause disruption of the blood-brain barrier (BBB) and to permit the transit of one or more brain-derived biomarkers into the blood, with a detection agent to detect the presence, absence or level of the one or more brain-derived biomarkers, optionally wherein the human patient has been suspected of having the disease or disorder of the brain or at risk for developing a disease or disorder of the brain; and (b) determining subtype or mutational basis of the disease or disorder based on step (a).

In embodiments, there is provided a method in which patient's tumor sample is characterized for somatic mutations that are specific to the patient's tumor to establish a Tumor Mutational Burden (TMB). In embodiments, a higher TMB indicates increased likelihood that the tumor will respond to immunotherapy. In embodiments, a TMB above a particular threshold, and/or presence of certain mutations, is used as a companion diagnostic for selecting therapy (e.g., immunotherapy and/or other targeted therapies). In embodiments, present methods allow for the creation of a patient-specific signature (*e.g.,* that is or comprises one or more somatic mutations, or a set thereof, and/or a particular TMB level) that can be detected in the blood and, in some embodiments, tracked over time. In embodiments, a higher level of somatic mutation found in blood as described herein indicates a higher tumor burden (*e.g.,* larger tumor size and/or greater number of tumors) in the subject. In embodiments, a lower level of somatic mutation found in blood as described herein indicates a lower tumor burden (*e.g.,* smaller tumor size and/or fewer tumors).

In embodiments, present technologies avoid false negatives that are resultant from the BBB which normally blocks the transit of biomarkers into the bloodstream.

In embodiments, provided technologies may be used, for example, to track minimal residual disease status, early recurrence, and/or to monitor response to treatment, for example by detecting a determined signature (*e.g.,* a mutation signature, such as a patient-specific signature as described herein, or another appropriate tumor signature determined in accordance with the present disclosure and/or through use of other technologies). In embodiments, if a tumor is progressing, a signature (*e.g.* a number of mutations and/or presence of particular mutations) increases. In embodiments, if a tumor is regressing (*e.g.,* in response to treatment), a signature (*e.g.* a number of mutations and/or presence of particular mutations) clears from the bloodstream, *i.e.* decreases. If a tumor is pseudo-progressing (*e.g.,* if immune inflammation is creating an appearance on imaging of tumor growth without actual tumor growth, and in some cases despite good treatment response) the signature does not increase. Those skilled in the art, reading the present disclosure, will appreciate that the same workflow can be utilized for a variety of types of signatures - *e.g.,* those including only somatic tumor mutations (and/or numbers thereof), and/or those including features such as methylation patterns and/or other epigenetic modifications in addition or as alternatives to somatic mutations.

### Brain-Derived Biomarkers

In embodiments, the present disclosure provides technologies relating to evaluation, e.g. without limitation, measuring a presence, absence, or level of one or more brain-derived biomarkers. In embodiments, provided technologies relate to detecting and/or quantifying or otherwise characterizing one or more brain-derived biomarkers.

In embodiments, detection of one or more brain-derived biomarker(s) as described herein is employed in decision-making regarding diagnosis, disease typing (*e.g.,* without limitation, determining disease subtype, mutation etiology, and the like), treatment selection, and/or theranosis, as described herein.

In embodiments, a brain-derived biomarker detected in accordance with the present disclosure circulates only in the cerebrospinal fluid in the absence of the focused ultrasound. In embodiments, "circulates" means that the biomarker is present at levels that are detectable in samples of the relevant biological fluid.

In embodiments, a brain-derived biomarker is contained within the brain and/or cerebrospinal fluid and is absent from the blood, without wishing to be bound by any particular theory, presumably due to the BBB. In embodiments, a brain-derived biomarker is contained within the brain and/or cerebrospinal fluid and prevented from transiting to the blood by the BBB. In embodiments, upon application of ultrasound energy as described herein to locally disrupt the BBB, a brain-derived biomarker transits from the cerebrospinal fluid to the blood via the disrupted BBB. In embodiments, a brain-derived biomarker is not substantially perturbed from a tumor cell by the ultrasound application or exposure. For instance, the ultrasound application or exposure allows for transit of free biomarker and does not alter the tissue from which the biomarker is derived. In embodiments, present methods do not target ultrasound at the tumor cells directly but target the tumor microenvironment (TME). In embodiments, present methods do not target ultrasound at tumor pathology, but other brain regions for region specific biomarker analysis, for example neurodegenerative disease, or other functional areas.

In embodiments, provided technologies target ultrasound at microbubbles, which act as an activator and/or interact with endothelial cells which open the BBB. In embodiments, provided technologies target ultrasound at the microbubbles within blood vessels.

In embodiments, a brain-derived biomarker is or comprises a disease or drug response biomarker.

In embodiments, a brain-derived biomarker is or comprises a nucleic acid.

In embodiments, a nucleic acid is or comprises DNA or RNA. In embodiments, a nucleic acid is or comprises cell-free DNA (cfDNA). In embodiments, a nucleic acid is or comprises circulating tumor DNA (ctDNA). In embodiments, a nucleic acid is or comprises a microRNA.

In embodiments, a brain derived biomarker is or comprises a protein. In embodiments, a brain derived biomarker is or comprises a cytokine.

In embodiments, a brain derived biomarker is or comprises a whole cell.

In embodiments, a brain derived biomarker is or comprises an extracellular vesicle. In embodiments, a extracellular vesicle is or comprises an exosome, ectosome, or large oncosome.

In embodiments, a brain-derived biomarker is or comprises a metabolite.

In embodiments, a brain-derived biomarker is or comprises a cancer biomarker. In embodiments, a cancer biomarker is selected from AFP, BCR-ABL, BRCA1/BRCA2, BRAF V600E, CA-125, CA19.9, CEA, EGFR, HER-2, KIT , PSA, PD-1, PD-L1, and S100. In embodiments, a cancer biomarker and cancer is selected from AFP (liver cancer), BCR-ABL (chronic myeloid leukemia), BRCA1/BRCA2 (breast/ovarian cancer), BRAF V600E (melanoma/colorectal cancer), CA-125 (ovarian cancer), CA19.9 (pancreatic cancer), CEA (colorectal cancer), EGFR (non-small-cell lung carcinoma), HER-2 (breast cancer), KIT (gastrointestinal stromal tumor), PSA (prostate specific antigen) (prostate cancer), and S100 (melanoma). In embodiments, a cancer biomarker and cancer is selected from mutations on genes KRAS, p53, EGFR, erbB2 for colorectal, esophageal, liver, and pancreatic cancer; mutations of genes BRCA1 and BRCA2 for breast and ovarian cancer; hypermethylation of MYOD1, CDH1, and CDH13 for cervical cancer; and hypermethylation of p16, p14, and RB1, for oral cancer In embodiments, a cancer biomarker and cancer is abnormal methylation of tumor suppressor genes p16, CDKN2B, and p14ARF for brain cancer.

In embodiments, a cancer biomarker is or comprises a Tumor Mutation Burden (TMB) profile. In embodiments, a detection of a high TMB (TMB-H) directs administration of an immunotherapeutic agent, optionally a checkpoint immunotherapy, optionally directed to PD-1 or PD-L1. In embodiments, a TMB-H is at least about 10 mutations/megabase based on a sequencing-based detection method, optionally FoundationOne CDx assay

In embodiments, a cancer biomarker is or comprises a glioblastoma biomarker. In embodiments, a glioblastoma biomarker is selected from IDH mutations, 1p19q deletion, MGMT promoter methylation, and EGFRvIII amplification. In embodiments, a IDH mutation is R132H.

In embodiments, a disease biomarker is or comprises an Alzheimer's disease biomarker. In embodiments, a Alzheimer's disease biomarker is selected from amyloid beta protein, tau protein, APOE ε4 variant, APP mutation, PSEN1 mutations, and PSEN2 mutations, methylation signatures and other epigenetic biomarkers on the released brain region-specific cfDNA.

In embodiments, a disease biomarker is or comprises a Parkinson's disease biomarker. In embodiments, a Parkinson's disease biomarker is selected from alpha-synuclein and GBA mutations, methylation signatures and other epigenetic biomarkers on the released brain region-specific cfDNA.

### Disease or Disorder of the Brain

In embodiments, a disease or disorder of the brain is or comprises a cancer. In embodiments, a cancer is or comprises a metastatic tumor in the brain. In embodiments, a metastatic tumor in the brain originates in a breast, lung, skin (including melanoma), prostate gland, uterus, kidney or gastrointestinal tract. In embodiments, a tumor is or comprises a primary brain tumor. In embodiments, a primary brain tumor is glial. In embodiments, a glial tumor is one or more of astrocytoma, ependymoma, glioblastoma multiforme (GBM), medulloblastoma, and oligodendroglioma. In embodiments, a primary brain tumor is non-glial. In embodiments, a tumor is a posterior fossa tumor, optionally selected from a cerebellar tumor, pontine tumor, and medulloblastoma (primitive neuroectodermal tumor). In embodiments, a tumor is a cerebral hemisphere tumor. In embodiments, a cancer is or comprises a glioblastoma.

In embodiments, present method involves BBB disruption and liquid biopsy (*e.g.,* of a liquid other than a CNS liquid - *e.g.,* other than cerebrospinal fluid, and in particular of blood) with use of specific biomarkers, which may, for example, include one or more of proteins, cell free nucleic acid (*e.g.,* cfDNA and/or cfRNA), one or more epigenetic (*e.g.,* methylation) signatures, signatures of "hot" and "cold" tumors, *etc.* When combined with immunotherapy and/or immune priming adjuvant therapy, the method allows for the simultaneous detection of immune antigen release and immune response/antigen presentation to monitor and guide therapy.

In embodiments, provided methods, e.g. by virtue of directly acoustic energy to the TME and opening the BBB to systemic immune surveillance, provide for synergistic immune priming, therapeutic drug delivery, and/or region-specific immune monitoring.

In embodiments, present methods allow for classification of a patient or patient population as having "cold" or "hot" tumors. In embodiments, present methods allow for classification of a patient or patient population as having "cold" or "hot" tumors and directs treatment (e.g. those having "hot" tumors being administered systemic immunotherapies like checkpoint inhibitors while those having "cold tumor" not being administered systemic immunotherapies like checkpoint inhibitors and, optionally, receiving alternative treatment, for example directed at improving the immune response).

In embodiments, present methods provide serial BBB disruption for therapeutic reasons, optionally in combination with serial liquid biopsy to analyze release of region-specific and disease-specific biomarkers over time. In some embodiments, such biomarkers can include one or more of proteins and cell free DNA, including tumor-derived DNA, called circulating tumor DNA (ctDNA). ctDNA carries the mutational profile of a tumor, including, for example, somatic mutations and/or epigenetic modifications associated with (and in some cases found only in) the tumor, and this profile can be tracked in the blood in correlation with the amount of tumor present in the region. Absent technologies provided by the present disclosure, such data have typically not been diagnostic for brain tumors because the BBB limits transit of this signal and so it has not been possible to track it for these purposes and/or collection of such data has not provided useful, actionable information, *e.g.* due to high false negative rates. However, as described herein, with BBB disruption it becomes possible to track these mutations over time in the blood stream. Higher amounts of somatic tumor-specific mutations (and/or other tumor-associated biomarkers) in the bloodstream are associated with higher amounts of tumor in the region being targeted. Conversely, lower amounts of tumor-specific mutations (and/or other tumor-associated biomarkers) detectable in the blood indicate reduction in tumor size which may, for example, relate to the success of a therapy being delivered. Alternatively or additionally, rate of change (i.e., of increase or decrease) of tumor-associated biomarker(s) detectable in blood as described herein can provide useful information indicative of rate of tumor growth or shrinkage.

Monitoring of increase or decrease, or rate thereof, of tumor-associated biomarker(s) over time provides a way to monitor treatment response, in some cases offering earlier diagnosis of responders and non-responders, which in turn permits faster pivoting to alternative therapies in patients unlikely to receive a benefit before their tumor burden becomes too large, as can happen when awaiting repeated serial imaging alone. Alternatively or additionally, such monitoring can be used as a measure of progression (*e.g.* tumor growth) versus pseudo-progression (*e.g.* immune response giving the appearance of increased inflammation and increased size on neuroimaging yet actual underlying tumor response as measured by clearance of the associated ctDNA mutations). Still further alternatively or additionally, such measurements can be used in an adaptive manner to update treatment decisions such as increasing the dosing of immune priming acoustic energy, and/or adding higher doses of thermal and cavitation energy to increase the ability to turn the "cold" tumor "hot." In some embodiments, tumor-associated biomarkers (*e.g.*, region-specific biomarkers) can be analyzed for cytokine and other protein panel profiles, as well as for methylation profiles to aid in the diagnosis of transition from "cold" to "hot." In addition to adjusting immune priming doses, addition of therapeutics (including, *e.g*., high molecular weight therapeutics such as biologic therapeutics - for example antibodies and/or antibody-drug conjugates, specifically including cytotoxic antibody-drug conjugates) to the immune priming regimen can be done in personalized tailored way by monitoring the evolution of specific biomarker (*e.g.,* ctDNA) signals over time (*e.g.* increase in EGFR or Her2 leading to utilization of cytotoxic payloads tied to these antibodies aimed at these targets) with or without increasing anti-tumor acoustic energies and/or with or without systemically administered immunotherapies from cells to checkpoints to agonist/immune activating therapies.

In embodiments, a disease or disorder of the brain is or comprises a neurodegenerative disease. In embodiments, a neurodegenerative disease is or comprises Alzheimer's disease.

In embodiments, technologies disclosed herein allow tracking stages of Alzheimer's disease in a patient or patient population. In embodiments, methods disclosed herein allow tracking stages of Alzheimer's disease in a patient or patient population that is improved relative to tau PET and/or serial invasive CSF sampling. In embodiments, methods disclosed herein allow tracking of Braak stages, describing the gradual regional deposition of hyper phosphorylated tau protein over the course of the disease. Braak stage is strongly associated with cognitive impairment and is part of the hallmark pathological criteria for the diagnosis of AD. Braak stage I and stage II are usually clinically asymptomatic with tau accumulation in the medial temporal lobe (MTL) and transentorhinal region, followed by the entorhinal cortex in stage II. Clinical symptoms develop in stages III-IV. Stage III is marked by the gradual spread of NFT into the limbic structures including the amygdala and hippocampus, with stage IV noting spread to the thalamus and claustrum. In embodiments, present methods allow for detection of progression or lack of progression between stages. In embodiments, present methods allow detection of response to a treatment agent, *e.g.* if a human patient or patient population does not progress between the stages.

In embodiments, a neurodegenerative disease is or comprises Parkinson's disease. In embodiments, methods disclosed herein allow for early diagnosis and treatment of Parkinson's disease. α-Synuclein is a presynaptic neuronal protein that is linked genetically and neuropathologically to Parkinson's disease. Its aberrant cytotoxic aggregation causes neuronal death and the symptoms of Parkinson's disease occur when this process involves the substantia nigra. In embodiments, methods disclosed herein allow tracking of stages of disease, e.g. lesions initially occur in the medulla oblongata and dorsal motor nucleus of the glossopharyngeal and vagal nerves and anterior olfactory nucleus (Braak Stage 1). The disease process in the brain stem pursues an ascending course with little interindividual variation. Spread into the pontine tegmentum occurs in Braak Stage 2 and arrival in the midbrain occurs in Braak Stage 3, in particular in the pars compacta of the substantia nigra. Braak Stage 4 involves the basal prosencephalon and temporal mesocortex (trasentorhinal region), with Braak Stage 5 and 6 involving the neocortex, with high order sensory association and prefrontal areas. First order sensory association/premotor areas and primary sensory/motor fields then follow suit. Tracking the regional spread of the disease and targeting the toxicity conferred by these aggregations may lead to novel therapeutic strategies not only in PD, but also in other related neurodegenerative conditions called synucleinopathies. In embodiments, present methods allow for detection of progression or lack of progression between stages. In embodiments, present methods allow detection of response to a treatment agent, *e.g.* if a human patient or patient population does not progress between the stages.

In embodiments, a disease or disorder of the brain is an anxiety disorder, for example, selected from generalized anxiety disorders, social phobias, specific phobias, panic disorders, obsessive compulsive disorder (OCD) and post-traumatic stress disorder (PTSD).

In embodiments, a disease or disorder of the brain is or comprises addiction.

In embodiments, a disease or disorder of the brain is or comprises bipolar disorder.

In embodiments, a disease or disorder of the brain is or comprises a behavior disorder selected from oppositional defiant disorder (ODD), conduct disorder (CD) and attention deficit hyperactivity disorder (ADHD).

In embodiments, a disease or disorder of the brain is or comprises depression.

In embodiments, a disease or disorder of the brain is or comprises an eating disorder selected from anorexia, bulimia nervosa and other binge eating disorders.

In embodiments, a disease or disorder of the brain is or comprises epilepsy.

In embodiments, a disease or disorder of the brain is or comprises an autoimmune disease.

In embodiments, a disease or disorder of the brain is or comprises multiple sclerosis.

In embodiments, a disease or disorder of the brain is or comprises dementia or cognitive dysfunction.

In embodiments, a disease or disorder of the brain is or comprises concussion or other traumatic brain injury.

### Patient Characteristics

In embodiments, a human patient or patient population is afflicted with, or suspected to be afflicted with a disease or disorder of the brain or at risk for developing a disease or disorder of the brain, e.g. any one of those described herein.

In embodiments, a human patient or patient population is afflicted with, or suspected to be afflicted with a cancer or at risk for developing a cancer. In embodiments, a cancer is or comprises any one of those described herein.

In embodiments, a cancer is or comprises an intracranial tumor.

In embodiments, a cancer is or comprises a metastatic tumor in the brain. In embodiments, a metastatic tumor in the brain originates in a breast, lung, skin (including melanoma), prostate gland, uterus, kidney or gastrointestinal tract.

In embodiments, a tumor is or comprises a primary brain tumor. In embodiments, a primary brain tumor is glial. In embodiments, a glial tumor is one or more of astrocytoma, ependymoma, glioblastoma multiforme (GBM), medulloblastoma, and oligodendroglioma. In embodiments, a primary brain tumor is non-glial. In embodiments, a brain tumor is a posterior fossa tumor, optionally selected from a cerebellar tumor, pontine tumor, and medulloblastoma (primitive neuroectodermal tumor). In embodiments, a brain tumor is a cerebral hemisphere tumor. In embodiments, a cancer is a glioblastoma.

In embodiments, a human patient or patient population is afflicted with, or suspected to be afflicted with a neurodegenerative disease or at risk for developing a neurodegenerative disease. In embodiments, a neurodegenerative disease is or comprises Alzheimer's disease. In embodiments, a neurodegenerative disease is or comprises Parkinson's disease.

In embodiments, a human patient or patient population is healthy and the present methods find use in general monitoring of brain health.

### Features of the Ultrasound Beam/Application Thereof

Various embodiments of the present disclosure relate to disruption of the BBB through the application of an ultrasound beam across the cranium of the human patient or patient population.

In embodiments, the disruption, permeabilization or opening of the BBB is "transient", meaning, in embodiments, non-permanent or temporary disruption, permeabilization, or opening of the BBB.

In embodiments, an ultrasound beam is or comprises a focused ultrasound beam. In embodiments, an ultrasound beam is or comprises a guided ultrasound beam.

In embodiments, an ultrasound beam is or comprises a focused ultrasound beam which is narrow (*e.g.* about 1-10 mm, or about 1-5 mm, or about 1-3 mm, or about 1 mm, or about 2 mm, or about 3 mm, or about 4 mm, or about 5 mm, or about 6 mm, or about 7 mm, or about 8 mm, or about 9 mm, or about 10 mm) or wide (*e.g.* about 1-10 cm, or about 1-5 cm, or about 1-3 cm, or about 1 cm, or about 2 cm, or about 3 cm, or about 4 cm, or about 5 cm, or about 6 cm, or about 7 cm, or about 8 cm, or about 9 cm, or about 10 cm).

In embodiments, an ultrasound beam is or comprises a focused, magnetic resonance-guided ultrasound beam (MRgFUS). In embodiments, an ultrasound beam is or comprises a focused, computerized tomography (CT)-guided ultrasound beam. In embodiments, an ultrasound beam is or comprises a focused, positron emission tomography (PET)-guided ultrasound beam. In embodiments, an ultrasound beam is or comprises a focused, positron emission tomography (PET)-guided ultrasound beam. In embodiments, an ultrasound beam is or comprises a focused, stereotactically-navigated guided ultrasound beam based on registration to prior scan.

In embodiments, a focused ultrasound beam is applied directly to the human patient's cranium, *e.g.* using a helmet-shaped ultrasound transducer.

In embodiments, a focused ultrasound beam is applied at a center frequency of about 180 to about 230 kHz, *e.g.* about 220 kHz.

In embodiments, an ultrasound beam is applied at a maximum power of up to about 30 W, *e.g.* about 4 W to about 30 W, about 6 W to about 30 W, about 8 W to about 30 W, about 10 W to about 30 W, or about 10 W to about 20 W, or about 20 W to about 30 W.

In embodiments in which the ultrasound beam does not go through nominal skull, beam power is reduced and power range is about 0.5 W to about 15 W, *e.g.* about 0.5 W, or about 5 W, or about 10 W, or about 15 W.

In embodiments, an ultrasound beam is delivered in about 3 mm sonication subspots separated by about 2.5-3 mm for continuous and/or slightly-overlapping delivery of In embodiments, an ultrasound beam is 1 -2 V*s target dose, which is, optionally, monitored in real-time via measured Webers of magnetic flux, *i.e.* electromotive force of one Volt induced per second.

In embodiments, a treatment duration is at least about 10 minutes, or at least about 20 minutes or at least about 30 minutes or at least about 40 minutes or at least about 50 minutes or at least about 60 minutes or at least about 70 minutes or at least about 80 minutes, or at least about 90 minutes, or at least about 120 minutes, or at least about 150 minutes, or at least about 180 minutes. In embodiments, a treatment duration is about 100 minutes, or about 110 minutes, or about 120 minutes, or about 130 minutes, or about 140 minutes, or about 150 minutes, or about 160 minutes.

In embodiments, an ultrasound beam is applied for at least about 10 seconds, or at least about 20 seconds, or at least about 30 seconds, or at least about 40 seconds, or at least about 50 seconds, or at least about 60 seconds.

In embodiments, an ultrasound beam is applied for about 10 seconds, or about 20 seconds, or about 30 seconds, or about 40 seconds, or about 50 seconds, or about 60 seconds.

In embodiments, an ultrasound beam is applied in pulses.

In embodiments, an ultrasound beam is applied at a power of at least about 5W, or at least about 10 W, or at least about 15W, or at least about 20W, or at least about 25W.

In embodiments, an ultrasound beam is applied at a power of about 10W, or about 15W, or about 20W.

In embodiments, an ultrasound beam is applied via a computationally generating a protocol for targeting of at least one site, as described in WO 2019/002943.

In embodiments, present methods employ microbubble-enhanced ultrasound procedures that employ an ultrasound system, a monitoring system and a microbubble administration system as described in WO 2019/116097.

In embodiments, an ultrasound beam targets one or more of the frontal lobe, parietal lobe, temporal lobe, occipital lobe, and cerebellum. In embodiments, an ultrasound beam targets the supratentorial region of the brain. In embodiments, an ultrasound beam targets the infratentorial region of the brain. In embodiments, an ultrasound beam targets the insula. In embodiments, an ultrasound beam targets the brainstem. In embodiments, an ultrasound beam targets the pons.

In embodiments, an ultrasound beam targets the posterior fossa. In embodiments, an ultrasound beam targets the corticomedullary gray/white junction. In embodiments, an ultrasound beam targets the meninges.

In embodiments, present disclosure relates to real-time monitoring during treatment by *e.g.* acoustic response or MRI imaging. In embodiments, real-time monitoring allows for detection of safety and/or efficacy of the present methods.

### Contacting/Detecting/Detection Agents

In embodiments, a biomarker is detected within about 10 minutes to about 60 minutes, or about 20 minutes to about 40 minutes after completion of the BBB disruption. In embodiments, a biomarker is detected within about 10 minutes, or about 20 minutes, or about 30 minutes, or about 40 minutes, or about 50 minutes, or about 60 minutes after completion of the BBB disruption

In embodiments, detecting comprises measuring a presence, absence, or level of a epigenetic pattern or profile. In embodiments, detecting comprises measuring a presence, absence, or level of a methylation pattern or signature. In embodiments, detecting comprises chromatin immunoprecipitation (ChIP) or bisulfite modification. In embodiments, detecting comprises measuring a presence, absence, or level of a histone modification.

In embodiments, detecting comprises measuring a presence, absence, or level of a mutant version of the biomarker. In embodiments, detecting comprises measuring a presence, absence, or level of a tumor-associated mutation. In embodiments, detecting comprises measuring a presence, absence, or level of a mutational burden. In embodiments, detecting comprises measuring a presence, absence, or level of a polymorphism. In embodiments, detecting comprises measuring a presence, absence, or level of a SNPs. In embodiments, detecting comprises measuring a presence, absence, or level of a copy number aberration, optionally as compared to a reference or parental genome. In embodiments, detecting comprises measuring a presence, absence, or level of a microsatellite instability.

In embodiments, a detection agent is or comprises a primer or probe. In embodiments, a detection agent is or comprises labeled probe, optionally fluorescently or radiolabeled. In embodiments, detecting comprises a primer-based detection method. In embodiments, detecting comprises a probe-based detection method.

In embodiments, detecting comprises polymerase chain reaction (PCR), reverse transcriptase polymerase chain reaction (RT-PCR), quantitative PCR (qPCR), multiplex polymerase chain reaction, nested polymerase chain reaction, hot start polymerase chain reaction, long-range PCR, assembly polymerase chain reaction, asymmetric polymerase chain reaction, digital droplet PCT(ddPCR) or BEAMing (beads, emulsion, amplification, and magnetics, see, *e.g.* Z. Cheng et al., "An emulsion digital PCR quantitative method based on microbeads and micropillar array chip," 2017 19th International Conference on Solid-State Sensors, Actuators and Microsystems (TRANSDUCERS), 2017, pp. 575-578).

In embodiments, detecting comprises a recombinase polymerase amplification (RPA), a loop-mediated amplification (LAMP), or a helicase-dependent amplification (HDA).

In embodiments, detecting comprises RNA or DNA gel electrophoresis or Southern or Northern blotting.

In embodiments, detecting comprises a microarray-based assay.

In embodiments, detecting comprises a hybridization technique, optionally selected from solution hybridization, capillary hybridization, hybridization to nucleic acid arrays, optionally macroarrays, microarrays or high-density oligonucleotide arrays (Gene Chips).

In embodiments, detecting comprises DNA sequencing.

In embodiments, a detection agent is or comprises an antibody. In embodiments, detecting comprises an antibody-based detection method. In embodiments, detecting comprises immunohistochemical staining, western blotting, in-cell western, immunofluorescent staining, ELISA, and fluorescent activating cell sorting (FACS). In embodiments, a detection agent is or comprises an antibody specific for an epigenetic signature and/or nucleosome-protein adduct. In embodiments, a detection agent is or comprises an antibody specific for a mutant protein.

In embodiments, detecting comprises spectroscopy, optionally mass spectroscopy.

### Diagnosing

In embodiments, present methods, in part or in whole, relate to diagnosis.

In embodiments, diagnosing comprises prediction of disease onset or progression.

In embodiments, diagnosing comprises prediction or monitoring of progression or pseudoprogression.

In embodiments, diagnosing comprises prediction or monitoring of response to treatment.

In embodiments, diagnosing comprises measuring of minimal residual disease status.

In embodiments, diagnosing comprises prediction of recurrence.

In embodiments, a disease or disorder of the brain is a glioblastoma and the brain-derived biomarker is selected from IDH mutations, 1p19q deletion, MGMT promoter methylation, and EGFRvIII amplification.

In embodiments, a neurodegenerative disease is Alzheimer's disease and the brain-derived biomarker is selected from amyloid beta protein, tau protein, APOE ε4 variant, APP mutation, PSEN1 mutations, and PSEN2 mutations.

In embodiments, a neurodegenerative disease is Parkinson's disease and the brain-derived biomarker is selected from alpha-synuclein and GBA mutations.

### Microbubbles

The present disclosure relates, in part, to the present ultrasound applications in conjunction with microbubbles. Microbubbles are typically gas-filled, typically air or perfluorocarbon, and oscillate and vibrate when a sonic energy field is applied and may reflect ultrasound waves. This distinguishes the microbubbles from surrounding tissues. In embodiments, because gas bubbles in liquid lack stability and would therefore quickly dissolve, microbubbles are encapsulated with a solid shell. In embodiments, a shell is made from either a lipid or a protein. Materials having a hydrophilic outer layer to interact with the bloodstream and a hydrophobic inner layer to house the gas molecules are the most thermodynamically stable. In embodiments, air, sulfur hexafluoride, and perfluorocarbon gases all can serve as the composition of the microbubble interior.

In embodiments, present methods comprise administration of one or more microbubble compositions or the human patient or patient population has undergone administration with one or more microbubble compositions immediately before and/or during the application of the ultrasound beam.

In embodiments, one or more microbubble compositions are administered immediately before and/or during the application of the ultrasound beam.

In embodiments, one or more microbubble compositions are administered contemporaneously with the application of the ultrasound beam.

In embodiments, microbubble compositions comprise one or more lipid-based microspheres. In embodiments, microbubble compositions are perflutren lipid microspheres. In embodiments, microbubble compositions comprise (R)-hexadecanoic acid, 1-[(phosphonoxy)methyl]-1,2-ethanediyl ester, monosodium salt (DPPA); (R)-4-hydroxy-N,N,Ntrimethyl-10-oxo-7-[(1-oxohexadecyl)oxy]-3,4,9-trioxa-4-phosphapentacosan-1-aminium, 4-oxide, inner salt (DPPC); and (R)-α-[6-hydroxy-6-oxido-9-[(1-oxohexadecyl)oxy]-5,7,11-trioxa-2-aza-6-phosphahexacos-1-yl]-ω-methoxypoly(ox-1,2-ethanediyl), monosodium salt; (N-(methoxypolyethylene glycol 5000 carbamoyl)-1,2-dipalmitoyl-sn-glycero-3- phosphatidylethanolamine, monosodium salt, MPEG5000 DPPE). In embodiments, microbubble compositions are lipid-coated echogenic microbubbles filled with octafluoropropane gas. In embodiments, microbubble compositions comprise octafluoropropane encapsulated in an outer lipid shell comprising (R)-hexadecanoic acid, 1-[(phosphonoxy)methyl]-1,2-ethanediyl ester, monosodium salt (DPPA); (R)-4-hydroxy-N,N,Ntrimethyl-10-oxo-7-[(1-oxohexadecyl)oxy]-3,4,9-trioxa-4-phosphapentacosan-1-aminium, 4-oxide, inner salt (DPPC); and (R)-α-[6-hydroxy-6-oxido-9-[(1-oxohexadecyl)oxy]-5,7,11-trioxa-2-aza-6-phosphahexacos-1-yl]-ω-methoxypoly(ox-1,2-ethanediyl), monosodium salt; (N-(methoxypolyethylene glycol 5000 carbamoyl)-1,2-dipalmitoyl-sn-glycero-3-phosphatidylethanolamine, monosodium salt, MPEG5000 DPPE).

In embodiments, microbubble compositions are administered to the patient no more than 60, or 30, or 20, or 10 minutes before the application of the ultrasound beam.

In embodiments, microbubble compositions are administered to the patient throughout the method.

In embodiments, microbubble compositions are administered by systemic injection, bolus injection or slow diffusion injection. In embodiments, microbubble compositions are administered by systemic infusion. In embodiments, microbubble compositions are administered by continuous intravenous infusion. In embodiments, microbubble compositions are administered by continuous intravenous infusion of a mixture about 1 x 10¹⁰ to about 6 x 10¹⁰ microsphere in a carrier. In embodiments, microbubble compositions are administered by continuous intravenous infusion of a mixture about 1 x 10¹⁰ to about 6 x 10¹⁰ microsphere in a saline carrier. In embodiments, microbubble compositions are administered by continuous intravenous infusion of a mixture about 1 x 10¹⁰ to about 6 x 10¹⁰ microsphere in about 250 mL of saline carrier. In embodiments, microbubble compositions are administered by continuous intravenous infusion of about 5 x 10⁷ to about 3 x 10⁸/mL. In embodiments, microbubble compositions are continuously infused at a rate of about 1 to about 3 mL/minute during the application of the ultrasound beam.

In embodiments, present disclosure relates, in part, to the present ultrasound applications in conjunction with alternatives microbubbles, such as nanodroplets or nanoparticles, e.g. gold nanoparticles. Embodiments relating to microbubbles apply equally to nanodroplets or nanoparticles, *e.g.* gold nanoparticles.

### Ultrasound Targets

In embodiments, application of the ultrasound beam targets at least one region of the brain.

In embodiments, application of the ultrasound beam targets at least two regions of the brain.

In embodiments, application of the ultrasound beam targets at least three regions of the brain.

In embodiments, application of the ultrasound beam targets at least one, or two, or three regions of the brain contemporaneously.

### Samples

In embodiments, present methods involve a variety of biological samples. Certain embodiments above refer to blood or plasma samples, but, in embodiments, a sample of mucus or saliva may substitute for the blood or plasma. Certain embodiments above refer to blood or plasma samples, but, in embodiments, a sample of lymph may substitute for the blood or plasma.

In embodiments, a sample is not a CSF sample.

As used herein, the word "include," and its variants, is intended to be non-limiting, such that recitation of items in a list is not to the exclusion of other like items that may also be useful in the materials, compositions, devices, and methods of this technology. Similarly, the terms "can" and "may" and their variants are intended to be non-limiting, such that recitation that an embodiment can or may comprise certain elements or features does not exclude other embodiments of the present technology that do not contain those elements or features. Although the open-ended term "comprising," as a synonym of terms such as including, containing, or having, is used herein to describe and claim the disclosure, the present technology, or embodiments thereof, may alternatively be described using more limiting terms such as "consisting of" or "consisting essentially of" the recited ingredients.

Unless defined otherwise, all technical and scientific terms herein have the same meaning as commonly understood by one of ordinary skill in the art to which this disclosure belongs. Although any methods and materials, similar or equivalent to those described herein, can be used in the practice or testing of the present disclosure, the preferred methods and materials are described herein.

This disclosure is further illustrated by the following non-limiting examples.

### EXAMPLES

In the following examples, nine patients were treated in a prospective single-arm, open-label trial to investigate serial MRgFUS and adjuvant temozolomide combination in patients with glioblastoma. Blood samples were collected within the hours before and after sonication, with control samples from non-brain tumor patients undergoing BBB opening alone.

The BBB restricts transport both into and out of the brain, which can present complications both for delivery of agents (e.g., therapeutic and/or diagnostic agents) to the brain, and for release of agents *(e.g.,* disease markers, evidence of therapeutic benefit or lack thereof, *etc.)* from the brain. This restriction of release means, in part, that markers of brain activity and/or status (e.g., presence of disease) typically are not readily detected other than by direct sampling of the CNS.

Work described herein represents the first-in-human demonstration that MRgFUS opening of the BBB in glioblastoma patients results in detectable levels of marker(s) such as brain-derived proteins, neuron-derived extracellular vesicles, and cell-free DNA in systemic samples (e.g., blood).

Differential analysis confirms that detected cell-free DNA is brain-derived and disease-specific.

Thus, the present disclosure demonstrates that, focused ultrasound can achieve release of brain-derived biomarkers into systemic fluids, thereby permitting, for example, detection and/or characterization of disease presence, status, and/or response to therapy through analysis of non-CNS sample(s) *(e.g.,* blood, serum, lymph, *etc.).* The present Examples specifically demonstrate surprising effectiveness of MRgFUS applied to neuro-oncology patients to permit assessment of brain-derived markers (specifically including cfDNA) in blood. Thus, the present Examples demonstrates that MRgFUS permits established liquid biopsy technologies to be applied in neuroncological contexts.

The Examples herein demonstrate that MRgFUS acutely enhanced plasma cfDNA (2.6±1.2 fold, p<0.01, Wilcoxon signed-rank test), neuron-derived extracellular vesicles (3.2±1.9 fold, p<0.01), and brain specific protein S100b (1.4±0.2 fold, p<0.01). Further comparison of the cfDNA methylation profiles suggests a signature that is disease and post-BBB opening specific. The Examples herein further demonstrate that, after MRgFUS-mediated opening of the BBB, levels of mutant isocitrate dehydrogenase I (IDH1) also increased (in diseased, but nor normal subjects).

Collectively, the Examples herein demonstrate that MRgFUS mediated enrichment of -brain-derived biomarkers, demonstrating that this technology can render brain diseases amenable to various liquid biopsy *(e.g.,* blood, serum, *etc.)* assessments.

### Example 1: Large Volume BBB Opening of the Tumor and Peritumoral Regions

In this Example, nine patients with WHO grade IV glioblastoma ("GBM") were enrolled in a single-arm trial investigating serial MRgFUS BBBO to enhance the delivery of adjuvant temozolomide therapy. All patients had undergone surgical resection, some gross total resection, and concurrent chemoradiation. The status of molecular markers was collected from clinical pathology at the time of surgery (see Table 1, below). Notably, IDH1-R132H status by immunohistochemistry was wildtype in all except patient 6 (P6).

**Table 1. Patient demographics**

| **Patient** | **Age** | **Gender** | **Eloquent Location** | **Extent of resection** | **MGMT promoter** | **IDH1-R132H** |
|---|---|---|---|---|---|---|
| **P1** | 49 | F | Yes | GTR | NA | Wildtype |
| **P2** | 52 | M | No | GTR | Methylated | Wildtype |
| **P3** | 56 | F | No | GTR | Unmethylated | Wildtype |
| **P4** | 35 | M | Yes | STR | NA | Wildtype |
| **P5** | 56 | F | Yes | STR | Unmethylated | Wildtype |
| **P6** | 42 | F | No | GTR | NA | Mutation |
| **P7** | 40 | F | Yes | GTR | Unmethylated | Wildtype |
| **P8** | 36 | F | Yes | GTR | Unmethylated | Wildtype |
| **P9** | 68 | M | No | GTR | Methylated | Wildtype |

The MRgFUS procedures were performed using a hemispheric array device (ExAblate, InSightec), overlapping the first day of each five-day temozolomide course (FIG. 1A, FIG. 1B). Each regimen was prescribed monthly. In total, 38 procedures were performed, with average 7.8 ± 6.0 cm³ (range 0.8 - 23.1 cm³) of tissue treated within sonication time of 111 ± 39 minutes. In tailoring the target with MRI guidance, the sonication of peri-tumor or peri-cavity regions was prioritized, as well as regions with FLAIR hyperintensities, to improve temozolomide penetration to presumably abnormal tissue (FIG. 1C). Ultrasound delivery was automated based on real-time monitoring of the acoustic emissions.

Immediately following the procedure, T1-weighted MRI visualized additional areas of gadobutrol (604 Da) enhancement, indicative of successful BBBO. Decreases in the enhancement the next day in all cases demonstrate partial or complete restoration of the BBB permeability (FIG. 1D). Maps of intensity differences show the spatial distribution of BBBO to envelope the tumor, which was feasible even for large, deep-seated lesions (FIG. 1E). The MRgFUS treatments were overall well- tolerated in all patients without any serious adverse events (see Table 2 below).

**Table 2. MRgFUS related adverse events over a total of 38 procedures**

| **Adverse event** | **Severity** | **N (%)** |
|---|---|---|
| **Intra-procedure** | | |
| Headache | Mild to moderate | 8 (21%) |
| Presyncope | Mild to moderate | 2 (5%) |
| Nausea/vomiting | Mild to moderate | 5 (13%) |
| Agitation | Mild | 2 (5%) |
| Hypotension | Mild | 2 (5%) |

| **Post-procedure, transient** | | |
|---|---|---|
| Pin site tenderness or edema | Mild to moderate | 11 (29%) |
| T2* GRE hypointensity | Mild | 2 (5%) |

### Example 2: Transient BBB Opening Increases the Concentration of Liquid Biopsy Analytes

In this Example, blood samples were collected within three hours prior to the first sonication (*e.g.,* during patient preparation), and on average 34 minutes after the last sonication. Plasma cfDNA concentration was acutely elevated after BBBO by 2.6 ± 1.2-fold (from 7.0 ± 3.3 ng/ml to 16.3 ± 5.2 ng/ml of plasma, p < 0.01, FIG. 2A). Values from individual cycles are listed in Table 3 below.

**Table 3. Plasma cfDNA concentrations pre- and post-BBBO at individual cvcles.**

| | | **Sonication volume (cm³)** | **Pre-BBBO (ng/mL plasma)** | **Post-BBBO (ng/mL plasma)** |
|---|---|---|---|---|
| Patient 1 | | | | |
| | Cycle 1 | 3.2 | 10.9 | 13.1 |
| | Cycle 2 | 3.5 | 9.0 | 17.7 6.9 |
| | Cycle 3 | 3.5 | 8.0 | 23.3 |
| | Cycle 4 | 4.8 | 10.8 | |

| Patient 2 | | | | |
|---|---|---|---|---|
| | Cycle 1 | 3.5 | 3.9 | 4.6 |
| | Cycle 2 | 2.5 | 5.7 | 9.4 |
| | Cycle 3 | 3.2 | 3.1 | 6.9 |
| | Cycle 4 | 5.5 | 3.0 | 11.0 |
| | Cycle 5 | 5.1 | 3.7 | 12.8 |
| | Cycle 6 | 5.9 | 4.8 | 10.3 |

| Patient 3 | | | | |
|---|---|---|---|---|
| | Cycle 1 | 0.8 | 11.0 | 8.3 |
| | Cycle 2 | 2.0 | 7.7 | 29.6 |
| | Cycle 3 | 3.2 | 13.1 | 26.0 |
| | Cycle 4 | 3.9 | 7.9 | 28.7 |

| Patient 4 | | | | |
|---|---|---|---|---|
| | Cycle 1 | 3.9 | 2.4 | 5.2 |
| | Cycle 2 | 4.7 | 6.3 | 23.0 |
| | Cycle 3 | 6.3 | - | 23.7 |

| Patient 5 | | | | |
|---|---|---|---|---|
| | Cycle 1 | 7.5 | 5.3 | 8.5 |
| | Cycle 2 | 9.8 | 5.6 | 15.0 |
| | Cycle 3 | 6.7 | 6.6 | 9.5 |
| | Cycle 4 | 3.5 | 6.0 | 14.0 |

| Patient 6 | | | | |
|---|---|---|---|---|
| | Cycle 1 | 5.9 | 5.7 | 22.3 |
| | Cycle 2 | 6.3 | 3.3 | 22.7 |
| | Cycle 3 | 7.9 | 4.8 | 13.5 |
| | Cycle 4 | 8.7 | 3.8 | 15.9 |
| | Cycle 5 | 7.9 | 4.1 | 9.2 |
| | Cycle 6 | 12.1 | 5.8 | 9.2 |

| Patient 7 | | | | |
|---|---|---|---|---|
| | Cycle 1 | 6.3 | 7.2 | 21.8 |
| | Cycle 2 | 7.9 | 10.7 | 30.0 |
| | Cycle 3 | 7.9 | 5.0 | 13.3 |
| | Cycle 4 | 14.2 | 4.1 | 16.8 |

| Patient 8 | | | | |
|---|---|---|---|---|
| | Cycle 1 | 19.2 | 4.1 | 11.7 |
| | Cycle 2 | 21.2 | 3.8 | 12.2 |
| | Cycle 3 | 20.3 | 4.9 | 11.6 |
| | Cycle 4 | 23.1 | 3.8 | 7.1 |
| | Cycle 5 | 22.0 | 4.2 | 11.2 |

| Patient 9 | | | | |
|---|---|---|---|---|
| | Cycle 1 | 4.9 | 13.7 | 17.1 |
| | Cycle 2 | 6.2 | 13.2 | 28.8 |

Up to a 7-fold increase in yield was measured. The cfDNA enhancement was consistently observed longitudinally through the adjuvant temozolomide cycles (FIG. 2B). To assess the quality and fragmentation pattern of the cfDNA, DNA electrophoresis was performed on one paired samples from each patient, and the results showed the 0 to 280 bp fragments increased from 4.9 ± 3.9 to 17.0 ± 14.9 ng/ml of plasma (p < 0.01) (FIG. 2C, FIG. 5).

MRgFUS-induced cfDNA increase appeared to share a positive correlation with treated volume (Spearman's correlation 0.33, p = 0.04, FIG. 2D), and a weaker negative correlation with time elapsed from sonication (Spearman's correlation -0.22, p = 0.20, FIG. 6). The level of cfDNA enhancement in two patients with subtotal resection was not different from the others (FIG. 7A), but there was a trend of greater enhancement with tumor progression (FIG. 7B).

The Examples herein demonstrated that a transient BBBO increases brain-derived biomarkers, specifically neuron-derived extracellular vesicles ("ndEV") and S100 calcium-binding protein B (S100b). The latter is primarily expressed by astrocytes. The former is robustly and specifically marked by NCAM and L1CAM surface proteins, and provides a diagnostic platform that can dynamically reflect and track neuropathological changes *in vivo.* From one randomly selected cycle from each patient, a 3.2 ± 1.9-fold (p < 0.01, FIG. 2E) increase was observed in double-positive NCAM and L1CAM particles using nanoscale flow cytometry (FIG. 8), and a 1.4 ± 0.2-fold (p < 0.01, FIG. 2F) in S100b.

### Example 3: Post-BBBO cfDNA Have Distinctive Disease-Specific Signature

DNA methylation profiling is a useful tool for classification of CNS tumors and their subtypes. In this Example, unsupervised classification of methylation microarray data of pre- and post-BBBO cfDNA shows a clear separation of the two groups by principle component 1, explaining for 25% of the variability (FIG. 3A). It was necessary to pool pre-BBBO samples collected from multiple cycles due to low cfDNA yield, while it was feasible to perform post-BBBO analysis on single sessions. Direct comparison of the beta values found 95% (330 of 346) of the differentially methylated probes in the post-BBBO samples were hypomethylated compared to pre-BBBO, consistent with a cancer signature (FIG. 9).

A gene set enrichment analysis was performed on the group of hypomethylated probes using g: Profiler. Based on protein expression data from the Human Protein Atlas, the set was significantly enriched for glia and neuron as well as immunologic cells (FIG. 3B). Of biological processes, enriched terms consisted of immune activation, vesicle-mediated transport, regulation of cell communication and DNA-binding transcription factor activity.

To determine whether the methylation signature of post-BBBO samples was unique to GBM patients, the experiments incorporated control plasma samples provided by two non-brain tumor patients with a history of Alzheimer's undergoing MRgFUS BBBO alone. Principle component analysis showed a clustering consistent with post-BBBO samples having differentiated signatures amongst patients with different neurological disorders (FIG. 3C).

IDH1 mutational status is an important molecular classification and prognostication factor for high-grade gliomas. Only one GBM tumor out of nine was immunopositive for the R132H mutation. Targeted analysis of two different cycles with droplet digital PCR (ddPCR), a highly sensitive technique, found 3.5 and 5 IDH1-R132H mutant copies per 10 ng cfDNA, which is considered a positive readout. These measurements represent a 2 to 3-fold increase from 1.6 mutant copies measured in the same input pre-BBBO cfDNA. Negative controls, from two patients with IDH1 wildtype tumors post-BBBO, had in comparison 1.7 ± 0.1 mutant copies per 10 ng cfDNA.

The results disclosed herein are the first-in-human proof-of concept studies documenting that MRgFUS enriches the signal of circulating brain-derived biomarkers, demonstrating the ability of the technology to support liquid biopsy assessment (e.g., via blood samples) for the brain.

The BBB physically limits the quantity of tumor analytes in the circulation. The Examples herein demonstrate for the first time in human patients that transcranial low-frequency MRgFUS can enrich the signal of circulating brain-derived biomarkers, specifically proteins, cfDNA, and extracellular vesicles ("EVs"), to help overcome this limitation. The Examples herein demonstrate a significant increase in cfDNA yield post-MRgFUS BBBO that bear a disease-specific methylation signature unique to brain tumor patients after BBBO. The Examples herein demonstrate an increased signal in clinically actionable plasma IDH1-R132H mutant copies, *(e.g.,* in a single patient harboring the IDH1-R132H tumor mutation). The Examples herein demonstrate the practical feasibility of repeated large volume BBBO in patients over the longitudinal course of adjuvant chemotherapy, supporting the flexibility of the technology when combined with liquid biopsy for disease monitoring.

### Methods

The Examples included demonstrate, among other things, that focused ultrasound enabled liquid biopsy enriches the signal of circulating biomarkers in patients with brain tumors. The work described herein was part of a prospective single-arm, open-label trial designed to investigate the safety and feasibility of serial MRgFUS and adjuvant temozolomide combination in patients with WHO grade IV glioblastoma (GBM). Details of the inclusion and exclusion criteria can be found in Table 4, below.

**Table 4. Study Inclusion and exclusion criteria**

| **Inclusion** | **Exclusion** |
|---|---|
| • 18-80 years of age | • ≥ 25% increase in contrast enhancement of the lesion on MRI at time of enrollment relative to first postoperative MRI |
| • Underwent surgical resection | |
| • Pathology diagnosis of WHO grade IV glioblastoma | |
| | • Evidence of increasing intracranial pressure |
| • Completed concurrent temozolomide chemoradiation without complications and deemed eligible for maintenance temozolomide therapy | • Receiving bevacizumab therapy |
| | • Undergoing other concurrent experimental therapies *e.g*. chemotherapy wafer, viral therapy, immunotherapies were excluded |
| | • Recent (< 2 weeks) intracranial hemorrhage |
| • Karnofsky score > 70 | • Increased risk of bleeding, *e.g*. anticoagulation or antiplatelet therapy |
| • ASA 1-3 | • Contraindication to MRI, gadolinium-based contrast, and ultrasound contrast agent Definity^{®} |
| | • TIA within the last 1 month, documented cerebral infarction within the last 12 months |
| | • Cerebral or systemic vasculopathy |
| | • Severe hypertension, cardiac and pulmonary disorders |

Blood samples were collected from all patients for exploratory analysis. Non-brain tumor control samples were provided by the initial patients enrolled in the MRgFUS BBBO for Alzheimer's disease ("AD") study.

Cancer patients underwent the procedure on the first day of every cycle, approximately thirty minutes after ingesting oral temozolomide prescribed by the study neuro-oncologist (FIG. 1A). Procedures were performed with the ExAblate Neuro hemispheric device (InSightec, Israel) coupled with GE 3-Tesla MRI. The device consists of a hemi-spherical dome with 1024 individual 220 kHz transducer elements that enable precise transcranial MRgFUS delivery. Specifically, the treatment volumes were prescribed by the neurosurgeon by outlining any non-enhancing tumor and a 1-centimeter peritumoral non-enhancing margin on 4 mm interval axial MRIs (FIG. 1B, FIG. 1C). Each contour was then filled in by the device software with sonication points separated by 2.5 mm, with approximately 7 mm out-of-plane depth (FIG. 1C). Contours were kept to less than 20 spots. Ultrasound delivery was performed contour-by-contour in a serial fashion, and was automated using on real-time acoustic emission. In the case of control patients with AD, widespread regions including the hippocampus and parietal lobe were treated by targeting in a similar fashion. Upon completion of the procedure, patients were allowed to leave once meeting routine discharge criteria. Standard-of-care procedures (*e.g*. MRI) and RANO assessments continued as usual. The procedures ended for a patient (***i.e**.* study exit) when adjuvant TMZ was no longer indicated (e.g. progressive disease, or drug toxicity).

### T1-weighted MRI analysis

BBBO was assessed by contrast-enhanced T1-weighted MRIs, which was validated against histological markers of BBB integrity. Intensity difference maps were calculated from same and next day scans by brain extraction, rigid-body co-registration, masking to the region of interest, rescaling to 0 and 1, and subtraction (FMRIB software library v6.0, Advanced Normalization Tools v2.1).

### Blood collection and s100b measurement

For all patients, blood was collected immediately prior to, and following, each procedure in EDTA-coated tubes (BD, 366643). Within 30 minutes of collection, plasma tubes were centrifuged at 800 g for 10 minutes at 4 °C, then 12,000 g for 10 minutes at room temperature. Clarified supernatant was aliquoted into DNA Lo-Bind tubes (Eppendorf, 22431021) for storage at -80 °C. Sample for any analysis will have undergone at the most one freeze-thaw cycle. S100b protein levels were measured via enzyme-linked immunosorbent assay kit (Sigma-Aldrich, EZHS100B-33K) as per manufacturer instructions.

### cfDNA extraction

The MagMAX Cell-Free DNA Isolation kit (Applied Biosystems, A29319) was used to extract cfDNA. DNA concentration was measured by Qubit 3.0 Fluorometer with Qubit dsDNA HS Assay Kit (Invitrogen, Q32854), and the fragmentation pattern was assessed using the Agilent 2100 Bioanalyzer with Agilent High Sensitive DNA Kit, following manufacturer's instructions.

### Methylation profile analysis

100-200 ng of extracted cfDNA, which was necessarily pooled for pre-BBBO samples, were bisulfite-converted using EZ DNA Methylation kit (Zymo Research, D5002) following the manufacturer's protocol. Effective conversion was confirmed by qPCR amplification of converted and unconverted beta-actin (see Table 5, below).

**Table 5. Primers used for bisulfite-conversion quality control qPCR**

| **Converted beta-actin** | **Unconverted beta-actin** |
|---|---|
| forward: 5'-TGG TGA TGG AGG AGG TTT AGT AAG T-3' (SEQ ID NO: 1) | forward: 5'-TGG TGA TGG AGG AGG CTC AGC AAG T-3' (SEQ ID NO: 3) |
| reverse: 5'-AAC CAA TAA AAC CTA CTC CTC CCT TAA-3' (SEQ ID NO: 2) | reverse: 5'-AGC CAA TGG GAC CTG CTC CTC CCT TGA-3' (SEQ ID NO: 4) |
| | |

Bisulfite-converted DNA was evaluated for methylation profiling using Illumina MethylationEPIC 850k array (Illumina, USA). Preprocessing with background subtraction adjustment was performed using GenomeStudio (Illumina, USA). Raw data files were processed using the minifi package (Bioconductor) in R and normalized. Probes that overlap with known single nucleotide polymorphisms, and probes that map to X and Y chromosomes were filtered out. Methylation values were exported as beta values. For unsupervised clustering, we performed a principle component analysis on the top 100,000 most variably methylated probes based on mean absolute deviation. Furthermore, differentially methylated probes between pre- and post-BBBO samples were identified by an absolute difference of mean beta value >0.1 and FDR-corrected p < 0.05. Functional enrichment analysis was conducted using g: Profiler tool.

### Plasma extracellular vesicles

NCAM (CD56) and L1CAM (CD171) positive EVs were measured by mixing and incubating 20 µL of plasma with Alexa Fluor 647 mouse anti-human CD56 (2 µL, BD Biosciences #557711) and PE mouse anti-human CD171 (2 µL, BD Biosciences #564193) at room temperature. Isotype controls were prepared similarly with Alexa Fluor 647 mouse IgG1 κ isotype control (2 µL, BD Biosciences # 57714), and PE Mouse IgG2a κ isotype control (2 µL, BD Biosciences #553457). A plasma sample from one cycle in every patient (Table 6, below) was diluted by 1:30 in sterile medical grade water and measured in duplicates using a nanoscale flow cytometer (Apogee Flow Systems Inc. A60Micro-Plus) calibrated with Apogee calibration bead mix. Data analysis was performed using Apogee Histogram Software v2020.

**Table 6. The cycle from which plasma extracellular vesicles were measured in each patient.**

| **Patient** | **Cycle number** |
|---|---|
| 1 | 1 |
| 2 | 6 |
| 3 | 3 |
| 4 | - |
| 5 | 4 |
| 6 | 5 |
| 7 | 3 |
| 8 | 6 |
| 9 | 2 |

### Digital droplet PCR

Frequency of isocitrate dehydrogenase 1 (IDH1) R132H mutations in plasma-derived cfDNA was assessed using a Bio-Rad QX200 ddPCR system. The ddPCR reaction was performed in a 20 µl volume containing 10 ng of isolated plasma cfDNA or IDH1 R132H immunopositive tumor DNA (positive control), 10 µL of ddPCR Supermix for Probes (No dUTP; Bio-Rad, cat. #186-3023) and 1 µL of Human FAM IDH1 p.R132H c.395G>A ddPCR Mutation Detection Assay (Bio-Rad, Assay ID dHsaMDV2010055). After mixing by vortexing and a spin down, droplets were generated using a QX200 Droplet Generator. Droplets were pipetted into a 96-well PCR plate, which was sealed and cfDNA samples amplified in the C1000 Touch Thermal Cycler (Bio-Rad) using the manufacturer recommended protocol. The fluorescent signal in each droplet was read with a QX200 Droplet Reader. Mutant and wild-type droplets were quantified using QuantaSoft analysis software ver.1.7.4.0917 (Bio-Rad).

### Statistical analysis

Descriptive statistics were used to summarize results, but wherever possible all data points were presented. Pairwise data were compared using Wilcoxon signed-rank test, with an alpha of 0.05.

## Claims

1. A method of evaluating a subtype or mutational basis of a disease or disorder of the brain in a human patient, comprising:
(a) contacting a plasma sample obtained from the human patient, the human patient having been exposed to an ultrasound beam across the cranium to cause disruption of the blood-brain barrier (BBB) and to permit the transit of one or more brain-derived biomarkers into the blood, with a detection agent to detect the presence, absence or level of the one or more brain-derived biomarkers, optionally wherein the human patient has been suspected of having the disease or disorder of the brain or at risk for developing a disease or disorder of the brain; and
(b) determining subtype or mutational basis of the disease or disorder based on step (a).

2. The method of claim 1, wherein the brain-derived biomarker:
circulates in the cerebrospinal fluid in the absence of the ultrasound;
transits from the cerebrospinal fluid to the blood via the disrupted BBB;
is not perturbed from a tissue by the ultrasound application or exposure, and/or is a disease or drug response biomarker, optionally wherein:
the disease biomarker is a cancer biomarker, optionally wherein:
the cancer biomarker is selected from AFP, BCR-ABL, BRCA1/BRCA2, BRAF V600E, CA-125, CA19.9, CEA, EGFR, HER-2, KIT , PSA, PD-1, PD-L1, and S100;
the cancer biomarker and cancer is selected from AFP (liver cancer), BCR-ABL (chronic myeloid leukemia), BRCA1/BRCA2 (breast/ovarian cancer), BRAF V600E (melanoma/colorectal cancer), CA-125 (ovarian cancer), CA19.9 (pancreatic cancer), CEA (colorectal cancer), EGFR (non-small-cell lung carcinoma), HER-2 (breast cancer), KIT (gastrointestinal stromal tumor), PSA (prostate specific antigen) (prostate cancer), and S100 (melanoma);
the cancer biomarker and cancer is selected from mutations on genes KRAS, p53, EGFR, erbB2 for colorectal, esophageal, liver, and pancreatic cancer; mutations of genes BRCA1 and BRCA2 for breast and ovarian cancer; hypermethylation of MYOD1, CDH1, and CDH13 for cervical cancer; and hypermethylation of p16, p14, and RB1, for oral cancer; or
the cancer biomarker is a Tumor Mutation Burden (TMB) profile, optionally wherein the detection of a high TMB (TMB-H) directs administration of an immunotherapeutic agent, or optionally a checkpoint immunotherapy, optionally directed to PD-1 or PD-L1, optionally wherein the TMB-H is at least about 10 mutations/megabase based on a sequencing-based detection method, optionally FoundationOne CDx assay;
the disease biomarker is abnormal methylation of tumor suppressor genes p16, CDKN2B, and p14ARF for brain cancer;
the disease biomarker is a glioblastoma biomarker, optionally wherein:
the glioblastoma biomarker is selected from IDH mutations, 1p19q deletion, MGMT promoter methylation, and EGFRvIII amplification;
the disease biomarker is an Alzheimer's disease biomarker optionally wherein:
the Alzheimer's disease biomarker is selected from amyloid beta protein, tau protein, APOE ε4 variant, APP mutation, PSEN1 mutations, and PSEN2 mutations; or
the disease biomarker is a Parkinson's disease biomarker, optionally wherein:
the Parkinson's disease biomarker is selected from alpha-synuclein and GBA mutations.

3. The method of claim 1 or claim 2, wherein:
the brain-derived biomarker is a nucleic acid optionally wherein:
the nucleic acid is DNA or RNA,
the nucleic acid is cell-free DNA (cfDNA), or
the nucleic acid is a microRNA;
the brain derived biomarker is a protein, optionally wherein:
the brain derived biomarker is a cytokine;
the brain derived biomarker is a whole cell;
the brain derived biomarker is an extracellular vesicle, optionally wherein:
the extracellular vesicle is an exosome, ectosome, or large oncosome; or
the brain-derived biomarker is a metabolite.

4. The method of any one of claims 1-3, wherein the disease or disorder of the brain is a cancer, optionally wherein
the brain cancer is a posterior fossa tumor, optionally selected from a cerebellar tumor, pontine tumor, and medulloblastoma (primitive neuroectodermal tumor), or
the brain cancer is a cerebral hemisphere tumor
optionally wherein:
the cancer is a metastatic tumor in the brain, optionally wherein:
the metastatic tumor in the brain originates in a breast, lung, skin, prostate gland, uterus, kidney or gastrointestinal tract;
the cancer is a primary brain tumor, optionally wherein:
the primary brain tumor is glial, optionally wherein:
the glial tumor is one or more of astrocytoma, ependymoma, glioblastoma multiforme (GBM), medulloblastoma, and oligodendroglioma;
the primary brain tumor is non-glial; or
the cancer is a glioblastoma.

5. The method of any one of claims 1-3, wherein the disease or disorder of the brain is selected from a neurodegenerative disease; Alzheimer's disease; Parkinson's disease, an anxiety disorder selected from generalized anxiety disorders, social phobias, specific phobias, panic disorders, obsessive compulsive disorder (OCD) and post-traumatic stress disorder (PTSD); addiction; bipolar disorder; a behavior disorder selected from oppositional defiant disorder (ODD), conduct disorder (CD) and attention deficit hyperactivity disorder (ADHD); depression; an eating disorder selected from anorexia, bulimia nervosa and other binge eating disorders; epilepsy; an autoimmune disease; multiple sclerosis; dementia; and cognitive dysfunction, and/or
wherein the human patient is afflicted with a neurodegenerative disease, suspected to be afflicted with a neurodegenerative disease, or at risk for developing a neurodegenerative disease, optionally wherein the neurodegenerative disease is Alzheimer's disease or Parkinson's disease.

6. The method of any one of claims 1-4, wherein the human patient is afflicted with a cancer, suspected to be afflicted with a cancer, or at risk for developing a cancer, optionally wherein:
the cancer is a metastatic tumor in the brain, optionally wherein: the metastatic tumor in the brain originates in a breast, lung, skin, prostate gland, uterus, kidney or gastrointestinal tract;
the tumor is a primary brain tumor, optionally wherein:
the primary brain tumor is glial, optionally wherein:
the glial tumor is one or more of astrocytoma, ependymoma, glioblastoma multiforme (GBM), medulloblastoma, and oligodendroglioma, or
the primary brain tumor is non-glial;
optionally wherein:
the brain tumor is a posterior fossa tumor, optionally selected from a cerebellar tumor, pontine tumor, and medulloblastoma (primitive neuroectodermal tumor);
the brain tumor is a cerebral hemisphere tumor; or
the cancer is a glioblastoma.

7. The method of any one of claims 1-6, wherein the ultrasound beam is a focused, ultrasound beam; a guided ultrasound beam; a focused, magnetic resonance-guided ultrasound beam (MRgFUS); a focused, computerized tomography (CT) -guided ultrasound beam; a focused, positron emission tomography (PET)-guided ultrasound beam; and/or a focused, stereotactically-navigated guided ultrasound beam based on registration to prior scan, optionally, wherein the ultrasound beam has been applied directly to the human patient's cranium using a helmet-shaped ultrasound transducer.

8. The method of any one of claims 1-7, wherein the ultrasound beam has been applied:
at a center frequency of about 220 kHz;
at a maximum power of up to about 30 Watts;
for at least about 10 seconds, or at least about 20 seconds, or at least about 30 seconds, or at least about 40 seconds, or at least about 50 seconds, or at least about 60 seconds, optionally for about 10 seconds, or about 20 seconds, or about 30 seconds, or about 40 seconds, or about 50 seconds, or about 60 seconds;
in pulses; and/or
at a power of at least about 5W, or at least about 10 W, or at least about 15W, or at least about 20W, or at least about 25W, optionally at a power of about 10W, or about 15W, or about 20W.

9. The method of any one of claims 1-8, wherein the ultrasound beam targets one or more of the frontal lobe, parietal lobe, temporal lobe, occipital lobe, and cerebellum; the supratentorial region of the brain; the infratentorial region of the brain; the insula; the brainstem; the pons; the posterior fossa; the corticomedullary gray/white junction; and/or the meninges.

10. The method of any one of claims 1-9, wherein the determining comprises:
measuring a presence, absence, or level of an epigenetic pattern or profile;
measuring a presence, absence, or level of a methylation pattern or signature;
chromatin immunoprecipitation (ChIP) or bisulfite modification;
measuring a presence, absence, or level of a histone modification;
measuring a presence, absence, or level of a mutant version of the biomarker;
measuring a presence, absence, or level of a tumor-associated mutation;
measuring a presence, absence, or level of a mutational burden;
measuring a presence, absence, or level of a polymorphism;
measuring a presence, absence, or level of a SNPs;
measuring a presence, absence, or level of a copy number aberration, optionally as compared to a reference or parental genome;
measuring a presence, absence, or level of a microsatellite instability;
an primer-based detection method;
an probe-based detection method;
polymerase chain reaction (PCR), reverse transcriptase polymerase chain reaction (RT-PCR), quantitative PCR (qPCR), multiplex polymerase chain reaction, nested polymerase chain reaction, hot start polymerase chain reaction, long-range PCR, assembly polymerase chain reaction, asymmetric polymerase chain reaction, or digital droplet PCT;
a recombinase polymerase amplification (RPA), a loop-mediated amplification (LAMP), or a helicase-dependent amplification (HDA);
RNA or DNA gel electrophoresis or Southern or Northern blotting;
a microarray-based assay;
a hybridization technique, optionally selected from solution hybridization, capillary hybridization, hybridization to nucleic acid arrays, optionally macroarrays, microarrays or high-density oligonucleotide arrays (Gene Chips);
DNA sequencing;
an antibody-based detection method;
spectroscopy, optionally mass spectroscopy; and/or
immunohistochemical staining, western blotting, in-cell western, immunofluorescent staining, ELISA, and fluorescent activating cell sorting (FACS).

11. The method of any one of claims 1-10, wherein the detection agent is a primer or probe, optionally a labeled probe, optionally fluorescently or radiolabeled;
an antibody;
an antibody specific for an epigenetic signature and/or nucleosome-protein adduct; and/or
an antibody specific for a mutant protein.

12. The method of any one of claims 1-11, wherein the diagnosing comprises prediction of disease onset or progression, progression or pseudoprogression, response to treatment, measuring of minimal residual disease status, and/or prediction of recurrence.

13. The method of any one of claims 1-12, wherein:
the disease or disorder of the brain is a glioblastoma and the brain-derived biomarker is selected from IDH mutations, 1p19q deletion, MGMT promoter methylation, and EGFRvIII amplification;
the disease or disorder of the brain is Alzheimer's disease and the brain-derived biomarker is selected from amyloid beta protein, tau protein, APOE ε4 variant, APP mutation, PSEN1 mutations, and PSEN2 mutations; and/or
the disease or disorder of the brain is Parkinson's disease and the brain-derived biomarker is selected from alpha-synuclein and GBA mutations.

14. The method of any one of claims 1-13, wherein the human patient has undergone administration with one or more microbubble compositions immediately before and/or during the application of the ultrasound beam, optionally wherein:
the one or more microbubble compositions have been administered immediately before and/or during the application of the ultrasound beam, or contemporaneously with the application of the ultrasound beam;
the microbubble compositions comprise one or more lipid-based microspheres;
the microbubble compositions are perflutren lipid microspheres;
microbubble compositions comprise (R)-hexadecanoic acid, 1-[(phosphonoxy)methyl]-1,2-ethanediyl ester, monosodium salt (DPPA); (R)-4-hydroxy-N,N,Ntrimethyl-10-oxo-7-[(1-oxohexadecyl)oxy]-3,4,9-trioxa-4-phosphapentacosan-1-aminium, 4-oxide, inner salt (DPPC); and (R)-α-[6-hydroxy-6-oxido-9-[(1-oxohexadecyl)oxy]-5,7,11-trioxa-2-aza-6-phosphahexacos-1-yl]-ω-methoxypoly(ox-1,2-ethanediyl), monosodium salt; (N-(methoxypolyethylene glycol 5000 carbamoyl)-1,2-dipalmitoyl-sn-glycero-3-phosphatidylethanolamine, monosodium salt, MPEG5000 DPPE);
the microbubble compositions are lipid-coated echogenic microbubbles filled with octafluoropropane gas;
the microbubble compositions comprise octafluoropropane encapsulated in an outer lipid shell comprising (R)-hexadecanoic acid, 1-[(phosphonoxy)methyl]-1,2-ethanediyl ester, monosodium salt (DPPA); (R)-4-hydroxy-N,N,Ntrimethyl-10-oxo-7-[(1-oxohexadecyl)oxy]-3,4,9-trioxa-4-phosphapentacosan-1-aminium, 4-oxide, inner salt (DPPC); and (R)-α-[6-hydroxy-6-oxido-9-[(1-oxohexadecyl)oxy]-5,7,11-trioxa-2-aza-6-phosphahexacos-1-yl]-ω-methoxypoly(ox-1,2-ethanediyl), monosodium salt; (N-(methoxypolyethylene glycol 5000 carbamoyl)-1,2-dipalmitoyl-sn-glycero-3-phosphatidylethanolamine, monosodium salt, MPEG5000 DPPE);
the microbubble compositions have been administered to the patient no more than 60, or 30, or 20, or 10 minutes before the application of the ultrasound beam;
the microbubble compositions have been administered to the patient throughout the method;
the microbubble compositions have been administered by systemic injection, bolus injection or slow diffusion injection;
the microbubble compositions have been administered by systemic infusion;
the microbubble compositions have been administered by continuous intravenous infusion;
the microbubble compositions have been administered by continuous intravenous infusion of a mixture about 1 x 10¹⁰ to about 6 x 10¹⁰ microsphere in a carrier;
the microbubble compositions have been administered by continuous intravenous infusion of a mixture about 1 x 10¹⁰ to about 6 x 10¹⁰ microsphere in a saline carrier;
the microbubble compositions have been administered by continuous intravenous infusion of a mixture about 1 x 10¹⁰ to about 6 x 10¹⁰ microsphere in about 250 mL of saline carrier;
the microbubble compositions have been administered by continuous intravenous infusion of about 5 x 10⁷ to about 3 x 10⁸/mL; and/or
the microbubble compositions are continuously infused at a rate of about 1 to about 3 mL/minute during the application of the ultrasound beam.

15. The method of any one of claims 1-14, wherein the application of the ultrasound beam targets:
at least one region of the brain;
at least two regions of the brain;
at least three regions of the brain; and/or
at least one, or two, or three regions of the brain contemporaneously.

## Patentansprüche

1. Verfahren zum Bewerten eines Subtyps oder einer Mutationsbasis einer Krankheit oder Störung des Gehirns bei einem menschlichen Patienten, umfassend:
(a) Inkontaktbringen einer Plasmaprobe, die von dem menschlichen Patienten erhalten wurde, wobei der menschliche Patient einem Ultraschallstrahl über den Schädel ausgesetzt wurde, um eine Unterbrechung der Blut-Hirn-Schranke (BHS) zu verursachen und den Übergang eines oder mehrerer aus dem Gehirn stammender Biomarker in das Blut zu erlauben, mit einem Nachweismittel zum Nachweisen der Anwesenheit, Abwesenheit oder des Niveaus des einen oder der mehreren aus dem Gehirn stammenden Biomarker, wobei optional der menschliche Patient im Verdacht steht, eine Krankheit oder Störung des Gehirns oder ein Risiko für die Entwicklung einer Krankheit oder Störung des Gehirns aufzuweisen; und
(b) Bestimmen des Subtyps oder der Mutationsbasis der Krankheit oder Störung basierend auf Schritt (a).

2. Verfahren nach Anspruch 1, wobei der aus dem Gehirn stammende Biomarker:
in Abwesenheit von Ultraschall in der Zerebrospinalflüssigkeit zirkuliert;
über die unterbrochene BHS von der Zerebrospinalflüssigkeit ins Blut übergeht;
durch die Anwendung oder Exposition von Ultraschall nicht in einem Gewebe gestört wird und/oder
ein Biomarker für eine Krankheit oder Arzneimittelantwort ist, wobei optional:
der Krankheitsbiomarker ein Krebsbiomarker ist, wobei optional:
der Krebsbiomarker ausgewählt ist aus AFP, BCR-ABL, BRCA1/BRCA2, BRAF V600E, CA-125, CA19.9, CEA, EGFR, HER-2, KIT , PSA, PD-1, PD-L1 und S100;
der Krebsbiomarker und der Krebs ausgewählt sind aus AFP (Leberkrebs), BCR-ABL (chronische myeloische Leukämie), BRCA1/BRCA2 (Brust-/Eierstockkrebs), BRAF V600E (Melanom/Darmkrebs), CA-125 (Eierstockkrebs), CA19.9 (Bauchspeicheldrüsenkrebs), CEA (Darmkrebs), EGFR (nichtkleinzelliges Lungenkarzinom), HER-2 (Brustkrebs), KIT (gastrointestinaler Stromatumor), PSA (prostataspezifisches Antigen) (Prostatakrebs) und S100 (Melanom);
der Krebsbiomarker und der Krebs ausgewählt sind aus Mutationen der Gene KRAS, p53, EGFR, erbB2 für Darm-, Speiseröhren-, Leber- und Bauchspeicheldrüsenkrebs; Mutationen der Gene BRCA1 und BRCA2 für Brust- und Eierstockkrebs; Hypermethylierung von MYOD1, CDH1 und CDH13 für Gebärmutterhalskrebs; und Hypermethylierung von p16, p14 und RB1 für Mundhöhlenkrebs; oder der Krebsbiomarker ein Tumor-Mutationslast(TMB)-Profil ist, wobei optional die Erkennung eines hohen TMB (TMB-H) auf die Verabreichung eines Immuntherapeutikums oder optional einer Checkpoint-Immuntherapie gerichtet ist, die optional auf PD-1 oder PD-L1 gerichtet ist, wobei optional das TMB-H mindestens etwa 10 Mutationen/Megabase beträgt, basierend auf einem sequenzbasierten Nachweisverfahren, optional dem FoundationOne CDx-Assay;
der Krankheitsbiomarker eine abnormale Methylierung der Tumorsuppressorgene p16, CDKN2B und p14ARF für Gehirntumore ist;
der Krankheitsbiomarker ein Glioblastom-Biomarker ist, wobei optional:
der Glioblastom-Biomarker ausgewählt ist aus IDH-Mutationen, 1p19q-Deletion, MGMT-Promotor-Methylierung und EGFRvlll-Amplifikation;
der Krankheitsbiomarker ein Biomarker für die Alzheimer-Krankheit ist,
wobei optional:
der Biomarker für die Alzheimer-Krankheit ausgewählt ist aus Amyloid-beta-Protein, Tau-Protein, APOE-ε4-Variante, APP-Mutation, PSEN1-Mutationen und PSEN2-Mutationen; oder
der Krankheitsbiomarker ein Biomarker für die Parkinson-Krankheit ist, wobei optional:
der Biomarker für die Parkinson-Krankheit ausgewählt ist aus Alpha-Synuclein- und GBA-Mutationen.

3. Verfahren nach Anspruch 1 oder Anspruch 2, wobei:
der aus dem Gehirn stammende Biomarker eine Nukleinsäure ist, wobei optional:
die Nukleinsäure DNA oder RNA ist,
die Nukleinsäure zellfreie DNA (cfDNA) ist oder
die Nukleinsäure eine microRNA ist;
der aus dem Gehirn stammende Biomarker ein Protein ist, wobei optional:
der aus dem Gehirn stammende Biomarker ein Zytokin ist;
der aus dem Gehirn stammende Biomarker eine ganze Zelle ist;
der aus dem Gehirn stammende Biomarker ein extrazelluläres Bläschen ist, wobei optional:
das extrazelluläre Bläschen ein Exosom, Ektosom oder großes Onkosom ist; oder der aus dem Gehirn stammende Biomarker ein Metabolit ist.

4. Verfahren nach einem der Ansprüche 1 bis 3, wobei die Krankheit oder Störung des Gehirns ein Krebs ist, wobei optional
der Gehirntumor ein Tumor der Fossa posterior ist, optional ausgewählt aus einem Kleinhirntumor, einem Pontintumor und einem Medulloblastom (primitiver neuroektodermaler Tumor), oder
der Gehirntumor ein Großhirnhemisphärentumor ist
wobei optional:
der Krebs ein metastatischer Tumor im Gehirn ist, wobei optional:
der metastatische Tumor im Gehirn aus einer Brust, einer Lunge, der Haut, der Prostatadrüse, der Gebärmutter, einer Niere oder dem Magen-Darm-Trakt stammt;
der Krebs ein primärer Gehirntumor ist, wobei optional:
der primäre Gehirntumor glial ist, wobei optional:
der gliale Tumor einer oder mehrere der folgenden Tumoren ist: Astrozytom, Ependymom, Glioblastoma multiforme (GBM), Medulloblastom und Oligodendrogliom;
der primäre Gehirntumor nicht glial ist; oder
der Krebs ein Glioblastom ist.

5. Verfahren nach einem der Ansprüche 1 bis 3, wobei die Krankheit oder Störung des Gehirns ausgewählt ist aus einer neurodegenerativen Erkrankung; Alzheimer-Krankheit; Parkinson-Krankheit, einer Angststörung, ausgewählt aus generalisierten Angststörungen, sozialen Phobien, spezifischen Phobien, Panikstörungen, Zwangsstörungen (OCD) und posttraumatischen Belastungsstörungen (PTSD); Sucht; bipolaren Störungen; einer Verhaltensstörung, ausgewählt aus oppositioneller Trotzstörung (ODD), Verhaltensstörung (CD) und Aufmerksamkeitsdefizit-Hyperaktivitätsstörung (ADHS); Depression; eine Essstörung, ausgewählt aus Anorexie, Bulimia nervosa und anderen Binge-Eating-Störungen; Epilepsie; einer Autoimmunerkrankung; Multipler Sklerose; Demenz; und kognitiver Dysfunktion, und/oder
wobei der menschliche Patient an einer neurodegenerativen Erkrankung leidet, oder wobei bei ihm der Verdacht besteht, dass er an einer neurodegenerativen Erkrankung leidet, oder wobei bei ihm das Risiko besteht, dass er eine neurodegenerative Erkrankung entwickelt, wobei die neurodegenerative Erkrankung optional die Alzheimer-Krankheit oder die Parkinson-Krankheit ist.

6. Verfahren nach einem der Ansprüche 1 bis 4, wobei der menschliche Patient an einem Krebs leidet, bei ihm der Verdacht besteht, dass er an einem Krebs leidet, oder bei ihm das Risiko besteht, dass er einen Krebs entwickelt, wobei optional:
der Krebs ein metastatischer Tumor im Gehirn ist, wobei optional:
der metastatische Tumor im Gehirn aus einer Brust, einer Lunge, der Haut, der Prostatadrüse, der Gebärmutter, einer Niere oder dem Magen-Darm-Trakt stammt;
der Tumor ein primärer Gehirntumor ist, wobei optional:
der primäre Gehirntumor glial ist, wobei optional:
der gliale Tumor einer oder mehrere der folgenden Tumoren ist: Astrozytom, Ependymom, Glioblastoma multiforme (GBM), Medulloblastom und Oligodendrogliom, oder der primäre Gehirntumor nicht glial ist;
wobei optional:
der Gehirntumor ein Tumor der Fossa posterior ist, optional ausgewählt aus einem Kleinhirntumor,
einem Pontintumor und einem Medulloblastom (primitiver neuroektodermaler Tumor);
der Gehirntumor ein Großhirnhemisphärentumor ist; oder
der Krebs ein Glioblastom ist.

7. Verfahren nach einem der Ansprüche 1 bis 6, wobei der Ultraschallstrahl ein fokussierter Ultraschallstrahl; ein geführter Ultraschallstrahl; ein fokussierter, magnetresonanzgeführter Ultraschallstrahl (MRgFUS); ein fokussierter, computertomographie-(CT)-geführter Ultraschallstrahl; ein fokussierter, positronenemissionstomographie-(PET)-geführter Ultraschallstrahl; und/oder ein fokussierter, stereotaktisch navigierter geführter Ultraschallstrahl, basierend auf der Registrierung eines vorherigen Scans ist, wobei der Ultraschallstrahl mittels eines helmförmigen Ultraschallwandlers direkt auf dem Schädel des menschlichen Patienten angewendet wurde.

8. Verfahren nach einem der Ansprüche 1 bis 7, wobei der Ultraschallstrahl angewendet wurde:
bei einer Mittenfrequenz von etwa 220 kHz;
bei einer maximalen Leistung von bis zu etwa 30 Watt;
für mindesten etwa 10 Sekunden oder mindesten etwa 20 Sekunden oder mindesten etwa 30 Sekunden oder mindesten etwa 40 Sekunden oder mindesten etwa 50 Sekunden oder mindesten etwa 60 Sekunden,
optional für etwa 10 Sekunden oder etwa 20 Sekunden oder etwa 30 Sekunden oder etwa 40 Sekunden oder etwa 50 Sekunden oder etwa 60 Sekunden;
in Impulsen; und/oder
bei einer Leistung von mindesten etwa 5 W oder mindesten etwa 10 W oder mindesten etwa 15 W oder mindesten etwa 20 W oder mindesten etwa 25 W, optional bei einer Leistung von etwa 10 W oder etwa 15 W oder etwa 20 W.

9. Verfahren nach einem der Ansprüche 1 bis 8, wobei der Ultraschallstrahl auf einen oder mehrere der folgenden Bereiche abzielt: Frontallappen, Parietallappen, Temporallappen, Okzipitallappen und Kleinhirn; die supratentorielle Region des Gehirns; die infratentorielle Region des Gehirns; die Insula; den Hirnstamm; die Brücke; die Fossa posterior; die kortikomedulläre graue/weiße Verbindung und/oder die Hirnhäute.

10. Verfahren nach einem der Ansprüche 1 bis 9, wobei das Bestimmen umfasst:
Messen der Anwesenheit, Abwesenheit oder des Niveaus eines epigenetischen Musters oder Profils;
Messen der Anwesenheit, Abwesenheit oder des Niveaus eines Methylierungsmusters oder einer Methylierungssignatur;
Chromatin-Immunpräzipitation (ChIP) oder Bisulfit-Modifikation;
Messen der Anwesenheit, Abwesenheit oder des Niveaus einer Histonmodifikation;
Messen der Anwesenheit, Abwesenheit oder des Niveaus einer mutierten Version des Biomarkers;
Messen der Anwesenheit, Abwesenheit oder des Niveaus einer tumorassoziierten Mutation;
Messen der Anwesenheit, Abwesenheit oder des Niveaus einer Mutationslast;
Messen der Anwesenheit, Abwesenheit oder des Niveaus eines Polymorphismus;
Messen der Anwesenheit, Abwesenheit oder des Niveaus von SNPs;
Messen der Anwesenheit, Abwesenheit oder des Niveaus einer Kopienzahlabweichung, optional im Vergleich zu einem Referenz- oder elterlichen Genom;
Messen der Anwesenheit, Abwesenheit oder des Niveaus einer Mikrosatelliteninstabilität;
ein primerbasiertes Nachweisverfahren;
ein sondenbasiertes Nachweisverfahren;
Polymerasekettenreaktion (PCR), Reverse-Transkriptase-Polymerasekettenreaktion (RT-PCR), quantitative PCR (qPCR), Multiplex-Polymerasekettenreaktion, verschachtelte Polymerasekettenreaktion, Hot-Start-Polymerasekettenreaktion, Long-Range-PCR, Montage-Polymerasekettenreaktion, asymmetrische Polymerasekettenreaktion oder digitale Tröpfchen-PCT;
eine Rekombinase-Polymerase-Amplifikation (RPA), eine Loop-vermittelte Amplifikation (LAMP) oder eine Helikase-abhängige Amplifikation (HDA);
RNA- oder DNA-Gelelektrophorese oder Southern- oder Northern-Blotting;
einen mikroarraybasierten Assay;
eine Hybridisierungstechnik, optional ausgewählt aus Lösungshybridisierung, Kapillarhybridisierung, Hybridisierung mit Nukleinsäurearrays, optional Makroarrays, Mikroarrays oder hochdichten Oligonukleotidarrays (Genchips);
DNA-Sequenzierung;
ein antikörperbasiertes Nachweisverfahren;
Spektroskopie, optional Massenspektroskopie; und/oder
immunhistochemische Färbung, Western-Blotting, In-Cell Western, Immunfluoreszenzfärbung, ELISA und Fluoreszenzaktivierende Zellsortierung (FACS).

11. Verfahren nach einem der Ansprüche 1 bis 10, wobei das Nachweismittel ein Primer oder eine Sonde ist, optional eine markierte Sonde, optional fluoreszenz- oder radioaktiv markiert;
ein Antikörper ist;
ein Antikörper ist, der spezifisch für eine epigenetische Signatur und/oder ein Nukleosom-Protein-Addukt ist; und/oder ein Antikörper ist, der spezifisch für ein mutiertes Protein ist.

12. Verfahren nach einem der Ansprüche 1 bis 11, wobei die Diagnose die Vorhersage des Ausbruchs einer Krankheit oder der Progression einer Krankheit oder der Progression oder Pseudoprogression, des Ansprechens auf eine Behandlung, das Messen des minimalen Restkrankheitsstatus und/oder die Vorhersage eines Rezidivs umfasst.

13. Verfahren nach einem der Ansprüche 1 bis 12, wobei:
die Krankheit oder Störung des Gehirns ein Glioblastom ist und der aus dem Gehirn stammende Biomarker ausgewählt ist aus IDH-Mutationen, 1p19q-Deletion, MGMT-Promotor-Methylierung und EGFRvIII-Amplifikation;
die Krankheit oder Störung des Gehirns die Alzheimer-Krankheit ist und der aus dem Gehirn stammende Biomarker ausgewählt ist aus Amyloid-beta-Protein, Tau-Protein, APOE-ε4-Variante, APP-Mutation, PSEN1-Mutationen und PSEN2-Mutationen; und/oder
die Krankheit oder Störung des Gehirns die Parkinson-Krankheit ist und der aus dem Gehirn stammende Biomarker ausgewählt ist aus Alpha-Synuclein und GBA-Mutationen.

14. Verfahren nach einem der Ansprüche 1 bis 13, wobei der menschliche Patient unmittelbar vor und/oder während der Anwendung des Ultraschallstrahls der Verabreichung einer oder mehrerer Mikrobläschen-Zusammensetzungen unterzogen wurde, wobei optional:
die eine oder die mehreren Mikrobläschen-Zusammensetzungen unmittelbar vor und/oder während der Anwendung des Ultraschallstrahls oder gleichzeitig mit der Anwendung des Ultraschallstrahls verabreicht wurden;
die Mikrobläschen-Zusammensetzungen eine oder mehrere lipidbasierte Mikrokügelchen umfassen;
die Mikrobläschen-Zusammensetzungen Perflutren-Lipid-Mikrokügelchen sind;
Mikrobläschen-Zusammensetzungen(R)-Hexadecansäure, 1-[(Phosphonoxy)methyl]-1,2-ethandiylester, Mononatriumsalz (DPPA); (R)-4-Hydroxy-N,N,N-trimethyl-10-oxo-7-[(1-oxohexadecyl)oxy]-3,4,9-trioxa-4-phosphapentacosan-1-aminium, 4-Oxid, inneres Salz (DPPC); und (R)-α-[6-Hydroxy-6-oxido-9-[(1-oxohexadecyl)oxy]-5,7,11-trioxa-2-aza-6-phosphahexacos-1-yl]-ω-methoxypoly(ox-1,2-ethandiyl), Mononatriumsalz; (N-(Methoxypolyethylenglykol 5000 Carbamoyl)-1,2-dipalmitoyl-sn-glycero-3-phosphatidylethanolamin, Mononatriumsalz, MPEG5000 DPPE) umfassen;
die Mikrobläschen-Zusammensetzungen lipidbeschichtete echogene Mikrobläschen sind, die mit Octafluorpropan-Gas gefüllt sind.
die Mikrobläschen-Zusammensetzungen Octafluorpropan umfassen, verkapselt in einer äußeren Lipidhülle, umfassend (R)-Hexadecansäure, 1-[(Phosphonoxy)methyl]-1,2-ethandiylester, Mononatriumsalz (DPPA). (R)-4-Hydroxy-N,N,N-trimethyl-10-oxo-7-[(1-oxohexadecyl)oxy]-3,4,9-trioxa-4-phosphapentacosan-1-aminium, 4-Oxid, inneres Salz (DPPC); und (R)-α-[6-Hydroxy-6-oxido-9-[(1-oxohexadecyl)oxy]-5,7,11-trioxa-2-aza-6-phosphahexacos-1-yl]-ω-methoxypoly(ox-1,2-ethandiyl), Mononatriumsalz; (N-(Methoxypolyethylenglykol 5000 Carbamoyl)-1,2-dipalmitoyl-sn-glycero-3-phosphatidylethanolamin, Mononatriumsalz, MPEG5000 DPPE);
die Mikrobläschen-Zusammensetzungen dem Patienten nicht eher als 60, 30, 20 oder 10 Minuten vor der Anwendung des Ultraschallstrahls verabreicht wurden;
die Mikrobläschen-Zusammensetzungen dem Patienten während des gesamten Verfahrens verabreicht wurden;
die Mikrobläschen-Zusammensetzungen durch systemische Injektion, Bolusinjektion oder langsame Diffusionsinjektion verabreicht wurden;
die Mikrobläschen-Zusammensetzungen durch systemische Infusion verabreicht wurden;
die Mikrobläschen-Zusammensetzungen durch kontinuierliche intravenöse Infusion verabreicht wurden;
die Mikrobläschen-Zusammensetzungen durch kontinuierliche intravenöse Infusion eines Gemisches von etwa 1 x 10¹⁰ bis etwa 6 x 10¹⁰ Mikrokügelchen in einem Träger verabreicht wurden;
die Mikrobläschen-Zusammensetzungen wurden durch kontinuierliche intravenöse Infusion eines Gemisches von etwa 1 x 10¹⁰ bis etwa 6 x 10¹⁰ Mikrokügelchen in einem salzhaltigen Träger verabreicht wurden;
die Mikrobläschen-Zusammensetzungen durch kontinuierliche intravenöse Infusion eines Gemisches von etwa 1 x 10¹⁰ bis etwa 6 x 10¹⁰ Mikrokügelchen in etwa 250 ml eines salzhaltigen Trägers verabreicht wurden;
die Mikrobläschen-Zusammensetzungen durch kontinuierliche intravenöse Infusion von etwa 5 x 10⁷ bis etwa 3 x 10⁸/ml verabreicht wurden; und/oder
die Mikrobläschen-Zusammensetzungen während der Anwendung des Ultraschallstrahls kontinuierlich mit einer Geschwindigkeit von etwa 1 bis etwa 3 ml/Minute infundiert wurden.

15. Verfahren nach einem der Ansprüche 1 bis 14, wobei die Anwendung des Ultraschallstrahls auf Folgendes abzielt:
mindestens einen Bereich des Gehirns;
mindestens zwei Bereiche des Gehirns;
mindestens drei Bereiche des Gehirns; und/oder
mindestens einen, oder zwei, oder drei Bereiche des Gehirns gleichzeitig.

## Revendications

1. Procédé d'évaluation d'un sous-type ou d'une base mutationnelle d'une maladie ou d'un trouble du cerveau chez un patient humain, comprenant :
(a) la mise en contact d'un échantillon de plasma obtenu à partir du patient humain, le patient humain ayant été exposé à un faisceau d'ultrasons à travers le crâne pour provoquer une ouverture de la barrière hémato-encéphalique (BHE) et pour permettre le transit d'un ou plusieurs biomarqueurs dérivés du cerveau dans le sang, avec un agent de détection pour détecter la présence, l'absence ou le niveau desdits un ou plusieurs biomarqueurs dérivés du cerveau, dans lequel facultativement il y avait une suspicion de présence de la maladie ou du trouble du cerveau ou d'un risque de développer une maladie ou un trouble du cerveau chez le patient humain ; et
(b) la détermination du sous-type ou de la base mutationnelle de la maladie ou du trouble sur la base de l'étape (a).

2. Procédé selon la revendication 1, dans lequel le biomarqueur dérivé du cerveau :
circule dans le liquide cérébrospinal en l'absence d'ultrasons ;
transite du liquide cérébrospinal vers le sang via la BHE ouverte ;
n'est pas perturbé du fait d'un tissu par l'application d'ultrasons ou l'exposition aux ultrasons, et/ou
est un biomarqueur de réponse à une maladie ou à un médicament, dans lequel facultativement :
le biomarqueur de la maladie est un biomarqueur du cancer, dans lequel facultativement :
le biomarqueur du cancer est sélectionné parmi les biomarqueurs AFP, BCR-ABL, BRCA1/BRCA2, BRAF V600E, CA-125, CA19.9, CEA, EGFR, HER-2, KIT, PSA, PD-1, PD-L1 et S100 ;
le biomarqueur du cancer et le cancer sont sélectionnés parmi les biomarqueurs AFP (cancer du foie), BCR-ABL (leucémie myéloïde chronique), BRCA1/BRCA2 (cancer du sein/de l'ovaire), BRAF V600E (mélanome/cancer colorectal), CA-125 (cancer de l'ovaire), CA19.9 (cancer du pancréas), CEA (cancer colorectal), EGFR (carcinome pulmonaire non à petites cellules), HER-2 (cancer du sein), KIT (tumeur stromale gastro-intestinale), PSA (antigène prostatique spécifique) (cancer de la prostate) et S100 (mélanome) ;
le biomarqueur du cancer est sélectionné selon le cancer parmi les mutations des gènes KRAS, p53, EGFR, erbB2 pour le cancer colorectal, œsophagien, hépatique et pancréatique ; les mutations des gènes BRCA1 et BRCA2 pour le cancer du sein et de l'ovaire ; l'hyperméthylation des gènes MYOD1, CDH1 et CDH13 pour le cancer du col de l'utérus ; et l'hyperméthylation des gènes p16, p14 et RB1 pour le cancer de la bouche ; ou le biomarqueur du cancer est un profil de charge de mutation tumorale (TMB - Tumor Mutation Burden), dans lequel facultativement la détection d'une TMB élevée (TMB-H) demande l'administration d'un agent immunothérapeutique, ou facultativement d'une immunothérapie de point de contrôle, facultativement dirigée vers le gène PD-1 ou PD-L1, dans lequel facultativement la TMB-H est d'au moins environ 10 mutations/mégabase selon un procédé de détection basé sur le séquençage, facultativement le test FoundationOne CDx ;
le biomarqueur de la maladie est une méthylation anormale des gènes suppresseurs de tumeurs p16, CDKN2B et p14ARF pour le cancer du cerveau ;
le biomarqueur de la maladie est un biomarqueur du glioblastome, dans lequel facultativement :
le biomarqueur du glioblastome est sélectionné parmi les mutations des IDH, la délétion 1p19q, la méthylation du promoteur du gène MGMT, et l'amplification du gène EGFRvIII ;
le biomarqueur de la maladie est un biomarqueur de la maladie d'Alzheimer, dans lequel facultativement :
le biomarqueur de la maladie d'Alzheimer est sélectionné parmi la protéine bêta-amyloïde, la protéine tau, le variant APOEε4, une mutation du gène APP, les mutations du gène PSEN1 et les mutations du gène PSEN2 ; ou
le biomarqueur de la maladie est un biomarqueur de la maladie de Parkinson, dans lequel facultativement :
le biomarqueur de la maladie de Parkinson est sélectionné parmi les mutations de l'alpha-synucléine et de la GBA.

3. Procédé selon la revendication 1 ou 2, dans lequel :
le biomarqueur dérivé du cerveau est un acide nucléique, dans lequel facultativement :
l'acide nucléique est de l'ADN ou de l'ARN,
l'acide nucléique est de l'ADN acellulaire, ou
l'acide nucléique est un microARN ;
le biomarqueur dérivé du cerveau est une protéine, dans lequel facultativement :
le biomarqueur dérivé du cerveau est une cytokine ;
le biomarqueur dérivé du cerveau est une cellule entière ;
le biomarqueur dérivé du cerveau est une vésicule extracellulaire, dans lequel facultativement :
la vésicule extracellulaire est un exosome, un ectosome ou un grand oncosome ; ou le biomarqueur dérivé du cerveau est un métabolite.

4. Procédé selon l'une quelconque des revendications 1 à 3, dans lequel la maladie ou le trouble du cerveau est un cancer, dans lequel facultativement le cancer du cerveau est une tumeur de la fosse crânienne postérieure, facultativement sélectionnée parmi une tumeur du cervelet, une tumeur pontine et un médulloblastome (tumeur neuroectodermique primitive), ou
le cancer du cerveau est une tumeur de l'hémisphère cérébral,
dans lequel facultativement :
le cancer est une tumeur métastatique dans le cerveau, dans lequel facultativement :
la tumeur métastatique dans le cerveau provient d'un sein, d'un poumon, de la peau, de la prostate, de l'utérus, d'un rein ou de l'appareil digestif ;
le cancer est une tumeur cérébrale primaire, dans lequel facultativement :
la tumeur cérébrale primaire est gliale, dans lequel facultativement :
la tumeur gliale correspond à une ou plusieurs des formes suivantes : astrocytome, épendymome, glioblastome multiforme (GBM), médulloblastome et oligodendrogliome ;
la tumeur cérébrale primaire est non gliale ; ou
le cancer est un glioblastome.

5. Procédé selon l'une quelconque des revendications 1 à 3, dans lequel la maladie ou le trouble du cerveau est sélectionné parmi une maladie neurodégénérative ; la maladie d'Alzheimer ; la maladie de Parkinson ; un trouble anxieux sélectionné parmi les troubles anxieux généralisés, les phobies sociales, les phobies spécifiques, les troubles paniques, le trouble obsessionnel-compulsif (TOC) et le trouble de stress posttraumatique (TSPT) ; une addiction ; un trouble bipolaire ; un trouble du comportement sélectionné parmi le trouble oppositionnel avec provocation (TOP), le trouble des conduites (TC) et le trouble déficitaire de l'attention avec hyperactivité (TDAH) ; la dépression ; un trouble alimentaire sélectionné parmi l'anorexie, la boulimie nerveuse et d'autres troubles d'hyperphagie compulsive ; l'épilepsie ; une maladie auto-immune ; la sclérose en plaques ; la démence ; et un dysfonctionnement cognitif, et/ou
dans lequel le patient humain est atteint d'une maladie neurodégénérative, suspecté d'être atteint d'une maladie neurodégénérative, ou à risque de développer une maladie neurodégénérative, dans lequel facultativement la maladie neurodégénérative est la maladie d'Alzheimer ou la maladie de Parkinson.

6. Procédé selon l'une quelconque des revendications 1 à 4, dans lequel le patient humain est atteint d'un cancer, suspecté d'être atteint d'un cancer ou à risque de développer un cancer, dans lequel facultativement :
le cancer est une tumeur métastatique dans le cerveau, dans lequel facultativement :
la tumeur métastatique dans le cerveau provient d'un sein, d'un poumon, de la peau, de la prostate, de l'utérus, d'un rein ou de l'appareil digestif ;
la tumeur est une tumeur cérébrale primaire, dans lequel facultativement :
la tumeur cérébrale primaire est gliale, dans lequel facultativement :
la tumeur gliale correspond à une ou plusieurs des formes suivantes : astrocytome, épendymome, glioblastome multiforme (GBM), médulloblastome et oligodendrogliome, ou la tumeur cérébrale primaire est non gliale ;
dans lequel facultativement :
la tumeur cérébrale est une tumeur de la fosse crânienne postérieure, facultativement sélectionnée parmi une tumeur du cervelet,
une tumeur pontine et un médulloblastome (tumeur neuroectodermique primitive) ;
la tumeur cérébrale est une tumeur de l'hémisphère cérébral ; ou
le cancer est un glioblastome.

7. Procédé selon l'une quelconque des revendications 1 à 6, dans lequel le faisceau d'ultrasons est un faisceau d'ultrasons focalisé ; un faisceau d'ultrasons guidé ; un faisceau d'ultrasons focalisé guidé par résonance magnétique (MRgFUS - Magnetic Resonance-guided Focused UltraSound) ; un faisceau d'ultrasons focalisé guidé par tomodensitométrie (CT - Computed Tomography) ; un faisceau d'ultrasons focalisé guidé par tomographie par émission de positons (PET - Positron Emission Tomography) ; et/ou un faisceau d'ultrasons focalisé guidé par navigation stéréotaxique sur la base d'un enregistrement de balayage antérieur, dans lequel facultativement le faisceau d'ultrasons a été appliqué directement au crâne du patient humain à l'aide d'un transducteur d'ultrasons en forme de casque.

8. Procédé selon l'une quelconque des revendications 1 à 7, dans lequel le faisceau d'ultrasons a été appliqué :
à une fréquence centrale d'environ 220 kHz ;
à une puissance maximale allant jusqu'à environ 30 watts ;
pendant au moins environ 10 secondes, ou au moins environ 20 secondes, ou au moins environ 30 secondes, ou au moins environ 40 secondes, ou au moins environ 50 secondes, ou au moins environ 60 secondes, facultativement pendant environ 10 secondes, ou environ 20 secondes, ou environ 30 secondes, ou environ 40 secondes, ou environ 50 secondes, ou environ 60 secondes ;
par impulsions ; et/ou
à une puissance d'au moins environ 5 W, ou d'au moins environ 10 W, ou d'au moins environ 15 W, ou d'au moins environ 20 W, ou d'au moins environ 25 W, facultativement à une puissance d'environ 10 W, ou d'environ 15 W, ou d'environ 20 W.

9. Procédé selon l'une quelconque des revendications 1 à 8, dans lequel le faisceau d'ultrasons cible une ou plusieurs structures parmi le lobe frontal, le lobe pariétal, le lobe temporal, le lobe occipital et le cervelet ; la région supratentorielle du cerveau ; la région infratentorielle du cerveau ; le cortex insulaire ; le tronc cérébral ; le pont ; la fosse crânienne postérieure ; la jonction grise/blanche corticomédullaire ; et/ou les méninges.

10. Procédé selon l'une quelconque des revendications 1 à 9, dans lequel la détermination comprend :
la mesure de la présence, de l'absence ou du niveau d'un modèle ou d'un profil épigénétique ;
la mesure de la présence, de l'absence ou du niveau d'un modèle ou d'une signature de méthylation ;
une immunoprécipitation de la chromatine (ChIP - Chromatin ImmunoPrecipitation) ou une modification au bisulfite ;
la mesure de la présence, de l'absence ou du niveau d'une modification d'histone ;
la mesure de la présence, de l'absence ou du niveau d'une version mutante du biomarqueur ;
la mesure de la présence, de l'absence ou du niveau d'une mutation associée à une tumeur ;
la mesure de la présence, de l'absence ou du niveau d'une charge mutationnelle ;
la mesure de la présence, de l'absence ou du niveau d'un polymorphisme ;
la mesure de la présence, de l'absence ou du niveau d'un polymorphisme mononucléotidique (SNP - Single-Nucleotide Polymorphism) ;
la mesure de la présence, de l'absence ou du niveau d'une aberration du nombre de copies, facultativement par rapport à un génome de référence ou parental ;
la mesure de la présence, de l'absence ou du niveau d'une instabilité de microsatellite ;
un procédé de détection basé sur une amorce ;
un procédé de détection basé sur une sonde ;
une PCR (Polymerase Chain Reaction, ou réaction en chaîne par polymérase), une PCR à transcriptase inverse (RT-PCR - Reverse Transcriptase PCR), une PCR quantitative (qPCR), une PCR multiplex, une PCR nichée, une PCR à démarrage à chaud, une PCR à longue portée, une PCR d'assemblage, une PCR asymétrique, ou une PCR numérique en gouttelettes ;
une amplification par recombinase polymérase (RPA - Recombinase Polymerase Amplification), une amplification médiée par boucles (LAMP - Loop-Mediated Amplification), ou une amplification hélicase-dépendante (HDA - Helicase-Dependent Amplification) ;
une électrophorèse sur gel d'ARN ou d'ADN, ou un transfert d'ADN (Southern blot) ou d'ARN (northern blot) ;
une analyse sur microréseau ;
une technique d'hybridation, facultativement sélectionnée parmi une hybridation en solution, une hybridation capillaire, une hybridation sur réseaux d'acides nucléiques, facultativement des macroréseaux, des microréseaux ou des réseaux d'oligonucléotides à haute densité (biopuces) ;
un séquençage d'ADN ;
un procédé de détection basé sur les anticorps ;
une spectroscopie, facultativement une spectroscopie de masse ; et/ou
une coloration immunohistochimique, un transfert de protéines (western blot), un transfert de protéines sans lyse cellulaire, une coloration immunofluorescente, une analyse immuno-enzymatique ELISA (Enzyme-Linked Immunosorbent Assay), et une cytométrie en flux à fluorescence FACS (Fluorescence-Activated Cell Sorting).

11. Procédé selon l'une quelconque des revendications 1 à 10, dans lequel l'agent de détection est une amorce ou une sonde, facultativement une sonde marquée, facultativement marquée par fluorescence ou radiomarquée ;
un anticorps ;
un anticorps spécifique d'une signature épigénétique et/ou d'un adduit nucléosome-protéine ; et/ou un anticorps spécifique d'une protéine mutante.

12. Procédé selon l'une quelconque des revendications 1 à 11, dans lequel le diagnostic comprend la prédiction de l'apparition ou de la progression de la maladie, de la progression ou de la pseudoprogression de la maladie, de la réponse au traitement, la mesure de l'état résiduel minimal de la maladie et/ou la prédiction d'une récidive.

13. Procédé selon l'une quelconque des revendications 1 à 12, dans lequel :
la maladie ou le trouble du cerveau est un glioblastome et le biomarqueur dérivé du cerveau est sélectionné parmi les mutations des IDH, la délétion 1p19q, la méthylation du promoteur du gène MGMT et l'amplification du gène EGFRvIII ;
la maladie ou le trouble du cerveau est la maladie d'Alzheimer et le biomarqueur dérivé du cerveau est sélectionné parmi la protéine bêta-amyloïde, la protéine tau, le variant APOEε4, une mutation du gène APP, les mutations du gène PSEN1 et les mutations du gène PSEN2 ; et/ou
la maladie ou le trouble du cerveau est la maladie de Parkinson et le biomarqueur dérivé du cerveau est sélectionné parmi les mutations de l'alphasynucléine et de la GBA.

14. Procédé selon l'une quelconque des revendications 1 à 13, dans lequel le patient humain a subi l'administration d'une ou plusieurs compositions de microbulles immédiatement avant et/ou pendant l'application du faisceau d'ultrasons, dans lequel facultativement :
lesdites une ou plusieurs compositions de microbulles ont été administrées immédiatement avant et/ou pendant l'application du faisceau d'ultrasons, ou simultanément à l'application du faisceau d'ultrasons ;
les compositions de microbulles comprennent une ou plusieurs microsphères à base de lipides ;
les compositions de microbulles sont des microsphères lipidiques de perflutren ;
les compositions de microbulles comprennent l'acide (R)-hexadécanoïque, du 1-[(phosphonoxy)méthyl]-1,2-éthanediyle ester, du sel monosodique d'azoture de diphénylphosphoryle (DPPA) ; du sel interne de (R)-4-hydroxy-N,N,Ntriméthyl-10-oxo-7-[(1-oxohexadécyl)oxy]-3,4,9-trioxa-4-phosphapentacosane-1-aminium 4-oxyde (DPPC) ; et du sel monosodique de (R)-α-[6-hydroxy-6-oxydo-9-[(1-oxohexadécyl)oxy]-5,7,11-trioxa-2-aza-6-phosphahexacose-1-yl]-ω-méthoxypoly(ox-1,2-éthanediyle) ; (sel monosodique de N-(méthoxypolyéthylène glycol 5000 carbamoyl)-1,2-dipalmitoyl-sn-glycéro-3-phosphatidyléthanolamine, MPEG5000 DPPE) ;
les compositions de microbulles sont des microbulles échogènes enrobées de lipides et remplies de gaz octafluoropropane ;
les compositions de microbulles comprennent de l'octafluoropropane encapsulé dans une enveloppe lipidique externe comprenant du sel monosodique d'acide (R)-hexadécanoïque, 1-[(phosphonoxy)méthyl]-1,2-éthanediyle ester (DPPA) ; du sel interne de (R)-4-hydroxy-N,N,Ntriméthyl-10-oxo-7-[(1-oxohexadécyl)oxy]-3,4,9-trioxa-4-phosphapentacosane-1-aminium, 4-oxyde (DPPC); et du sel monosodique de (R)-α-[6-hydroxy-6-oxydo-9-[(1-oxohexadécyl)oxy]-5,7,11-trioxa-2-aza-6-phosphahexacose-1-yl]-ωméthoxypoly(ox-1,2-éthanediyle); (sel monosodique de N-(méthoxypolyéthylène glycol 5000 carbamoyl)-1,2-dipalmitoyl-sn-glycéro-3-phosphatidyléthanolamine, MPEG5000 DPPE) ;
les compositions de microbulles ont été administrées au patient au plus tard 60, ou 30, ou 20, ou 10 minutes avant l'application du faisceau d'ultrasons ;
les compositions de microbulles ont été administrées au patient tout au long du procédé ;
les compositions de microbulles ont été administrées par injection systémique, par injection en bolus ou par injection à diffusion lente ;
les compositions de microbulles ont été administrées par perfusion systémique ;
les compositions de microbulles ont été administrées par perfusion intraveineuse continue ;
les compositions de microbulles ont été administrées par perfusion intraveineuse continue d'un mélange d'environ 1 × 10¹⁰ à environ 6 × 10¹⁰ microsphères dans un support ;
les compositions de microbulles ont été administrées par perfusion intraveineuse continue d'un mélange d'environ 1 × 10¹⁰ à environ 6 × 10¹⁰ microsphères dans un support salin ;
les compositions de microbulles ont été administrées par perfusion intraveineuse continue d'un mélange d'environ 1 × 10¹⁰ à environ 6 × 10¹⁰ microsphères dans environ 250 mL de support salin ;
les compositions de microbulles ont été administrées par perfusion intraveineuse continue d'environ 5 x 10⁷ à environ 3 × 10⁸/mL ; et/ou
les compositions de microbulles sont perfusées en continu à un débit d'environ 1 à environ 3 mL/minute pendant l'application du faisceau d'ultrasons.

15. Procédé selon l'une quelconque des revendications 1 à 14, dans lequel l'application du faisceau d'ultrasons cible :
au moins une région du cerveau ;
au moins deux régions du cerveau ;
au moins trois régions du cerveau ; et/ou
au moins une, ou deux, ou trois régions du cerveau simultanément.
